# EUROPEAN PATENT APPLICATION

(11) **EP 4 821 946 A1**
(43) Date of publication of application: **14.10.2026**
(21) Application number: 24899950.0
(22) Date of filing: 06.12.2024
(51) Int. Cl.: C07K 16/28, A61K 39/395, A61P 35/00, G01N 33/574

(54) **ANTI-CANINE PD-1 ANTIBODY AND USE THEREOF**

(30) Priority: 06.12.2023 CN 202311658628
(71) Applicant: Beijing Vjtbio Co., Ltd., Beijing 100094 (CN); Zhejiang VJTBio Co., Ltd., Taizhou, Zhejiang 317300 (CN)
(72) Inventor: LUO, Haoshu, Beijing 100094 (CN); TAN, Zemin, Beijing 100094 (CN); YANG, Youqiao, Beijing 100094 (CN); WU, Bingchun, Beijing 100094 (CN)
(74) Representative: Mewburn Ellis LLP
(86) International application number: PCT/CN2024/137348
(87) International publication number: WO 2025/119317

(57) **Abstract**

The present invention relates to the field of antibodies, and discloses an anti-canine PD-1 antibody and its use in preventing and treating mammalian tumors, especially use in preventing and treating canine tumors. The anti-canine PD-1 antibody with high affinity and high activity can block the binding of canine PD-1 to its ligand, so as to achieve the purpose of treating canine tumor diseases.

## Description

### Technical Field

The present invention relates to the technical field of antibodies, in particular to an anti-canine PD-1 antibody and use thereof.

### Background Art

Tumors are common and frequently-occurring diseases in humans and animals, in which canine malignant tumors have become the main cause of death in dogs. Relevant research data show that one out of every four dogs is diagnosed with a tumor, and the proportion of dogs over 10 years old suffering from tumors is even as high as 50% or more. At present, the clinical treatment means for canine tumors mainly comprises surgery, chemotherapy and radiotherapy. Common chemotherapeutic drugs comprise cyclophosphamide, doxorubicin, vincristine, prednisolone and the like, which have disadvantages such as poor tumor specificity and high toxic and side effects. Immunotherapy is an emerging treatment means, which may be effective and has few side effects for patients with poor response to chemotherapy. The research and application of immunotherapy has made a major breakthrough in human tumors, and it is of great significance to promote and apply immunotherapy to animal tumors.

Programmed cell death protein 1 (PD-1), as a type I transmembrane glycoprotein of about 55 kDa, belongs to the CD28 receptor superfamily, and is mainly expressed on the surface of T cells, B lymphocytes and activated macrophages. PD-1 proteins have two ligands: PD-L1 and PD-L2. Under normal physiological conditions, the binding of PD-1 to PD-L1/PD-L2 may inhibit the activation of T cells, thereby protecting the body from the attack of the autoimmune system; however, a variety of solid tumors and some hematologic tumors, including melanoma, breast cancer, various tumors of the digestive system, lymphoma, leukemia and other cancer cells, also highly express PD-L1. The PD-L1 on the tumor cell membrane binds to the PD-1 on T cells, inhibiting the activation of T cells, thereby successfully evading the recognition and attack of the body's immune system, and achieving the immune escape of tumor cells. Furthermore, the expression of PD-L1 on tumor cells was found to be associated with poor prognosis in multiple tumor types. Therefore, blocking the binding of PD-1 to PD-L1 is an idea of tumor immunotherapy.

### Summary of the Invention

In view of the shortcomings of the prior art, the present invention aims to provide a high-affinity and high-activity anti-canine PD-1 antibody, which can block the binding of canine PD-1 to its ligand, so as to achieve the purpose of treating canine tumor diseases.

In order to achieve the above objective of the invention, the present invention adopts the technical solutions as follows.

### A first aspect of the present invention provides CDR sequences of an antibody or antigen-binding fragment thereof capable of binding to canine PD-1.

The antibody or antigen-binding fragment thereof capable of binding to canine PD-1 comprises CDRs 1, 2 and 3 of a heavy chain variable region and/or CDRs 1, 2 and 3 of a light chain variable region.

According to an embodiment of the present invention, the antibody or antigen-binding fragment thereof capable of binding to canine PD-1 comprises a CDR sequence selected from at least one of the following CDR sequences or an amino acid sequence having at least 85% identity thereto: CDR sequences of the heavy chain variable region selected from SEQ ID NOs: 2-4, 8-11 and 14, and CDR sequences of the light chain variable region selected from SEQ ID NOs: 5-7, 12-13 and 15.

It is worth noting that in the above disclosure, the scope of protection is all possible CDR sequences within the above sequence range defined by the variable region, wherein the above CDRs 1-3 of the heavy and light chain variable regions may be obtained by different numbering systems in the prior art, including but not limited to Kabat, Chothia, IMGT, North, AbM, Contact and the like, and CDRs determined using other known or unknown numbering systems are also within the scope of protection of the present application. In addition, the CDR sequences predicted by different databases (such as different commercial, non-commercial, scientific research databases and the like for predicting CDR positions, including but not limited to abYsis database, IMGT database, TABS database, PatSnap BIO database, etc.) using numbering systems including but not limited to Kabat, Chothia, IMGT, North, AbM, Contact, etc. are also within the protection scope of the present application. CDRs, that are different to some extent, predicted by different databases using the same numbering system for the same antibody variable region sequence are also within the scope of protection of the present invention.

Further, the antibody or antigen-binding fragment thereof capable of binding to canine PD-1 comprises a heavy chain variable region and/or a light chain variable region having the following corresponding sequences or amino acid sequences having at least 85% identity thereto: a heavy chain variable region shown in SEQ ID NO: 16 and/or a light chain variable region shown in SEQ ID NO: 17;
or a heavy chain variable region shown in SEQ ID NO: 18 and/or a light chain variable region shown in SEQ ID NO: 21;
or a heavy chain variable region shown in SEQ ID NO: 18 and/or a light chain variable region shown in SEQ ID NO: 22;
or a heavy chain variable region shown in SEQ ID NO: 18 and/or a light chain variable region shown in SEQ ID NO: 23;
or a heavy chain variable region shown in SEQ ID NO: 19 and/or a light chain variable region shown in SEQ ID NO: 21;
or a heavy chain variable region shown in SEQ ID NO: 19 and/or a light chain variable region shown in SEQ ID NO: 22;
or a heavy chain variable region shown in SEQ ID NO: 19 and/or a light chain variable region shown in SEQ ID NO: 23;
or a heavy chain variable region shown in SEQ ID NO: 20 and/or a light chain variable region shown in SEQ ID NO: 21;
or a heavy chain variable region shown in SEQ ID NO: 20 and/or a light chain variable region shown in SEQ ID NO: 22;
or a heavy chain variable region shown in SEQ ID NO: 20 and/or a light chain variable region shown in SEQ ID NO: 23.

Further, the present invention provides an antibody or antigen-binding fragment thereof capable of binding to canine PD-1. According to an embodiment of the present invention, the antibody or antigen-binding fragment thereof capable of binding to canine PD-1 comprises a CDR sequence selected from at least one of the following CDR sequences or an amino acid sequence having at least 85% identity thereto: CDR sequences of the heavy chain variable region selected from SEQ ID NOs: 2-4, 8-11 and 14, and CDR sequences of the light chain variable region selected from SEQ ID NOs: 5-7, 12-13 and 15, or an amino acid sequence having at least 85% identity to the above sequence.

Further, the antibody or antigen-binding fragment thereof capable of binding to canine PD-1 comprises a CDR sequence selected from at least one of the following CDR sequences or an amino acid sequence having at least 85% identity thereto:
a heavy chain variable region CDR1 selected from any of SEQ ID NOs: 2, 8 and 14, a heavy chain variable region CDR2 selected from any of SEQ ID NOs: 3, 9 and 10, a heavy chain variable region CDR3 selected from any of SEQ ID NOs: 4 and 11; a light chain variable region CDR1 selected from any of SEQ ID NOs: 5 and 12, a light chain variable region CDR2 selected from any of SEQ ID NOs: 6, 13 and 15, and a light chain variable region CDR3 selected from SEQ ID NO: 7, or an amino acid sequence having at least 85% identity to the above sequence.

According to an embodiment of the present invention, the antibody or antigen-binding fragment thereof capable of binding to canine PD-1 comprises the following heavy chain variable region CDRs or amino acid sequences that can reach 85% or more identity thereto: heavy chain variable region CDR1, CDR2 and CDR3 sequences shown in amino acid sequences of SEQ ID NOs: 2, 3 and 4 respectively (the above sequences are obtained according to Kabat numbering system); heavy chain variable region CDR1, CDR2 and CDR3 sequences shown in amino acid sequences of SEQ ID NOs: 8, 9 and 4 respectively (the above sequences are obtained according to Chothia numbering system); or heavy chain variable region CDR1, CDR2 and CDR3 sequences shown in amino acid sequences of SEQ ID NOs: 14, 10 and 11 respectively (the above sequences are obtained according to IMGT numbering system).

According to an embodiment of the present invention, the antibody or antigen-binding fragment thereof capable of binding to canine PD-1 comprises the following light chain variable region CDRs or amino acid sequences that can reach 85% or more identity thereto: light chain variable region CDR1, CDR2 and CDR3 sequences shown in amino acid sequences of SEQ ID NOs: 5, 6 and 7 respectively (the above sequences are obtained according to Kabat or Chothia numbering system); light chain variable region CDR1, CDR2 and CDR3 sequences shown in amino acid sequences of SEQ ID NOs: 12, 13 and 7 respectively, or light chain variable region CDR1, CDR2 and CDR3 sequences shown in amino acid sequences of SEQ ID NOs: 12, 15 and 7 respectively (the above sequences are obtained according to IMGT numbering system and by analysis using different databases).

Specifically, the antibody or antigen-binding fragment thereof capable of binding to canine PD-1 comprises:
heavy chain variable region CDR1, CDR2 and CDR3 sequences shown in amino acid sequences of SEQ ID NOs: 2, 3 and 4, respectively, and light chain variable region CDR1, CDR2 and CDR3 sequences shown in amino acid sequences of SEQ ID NOs: 5, 6 and 7, respectively (the above sequences are obtained according to Kabat numbering system); or heavy chain variable region CDR1, CDR2 and CDR3 sequences shown in amino acid sequences of SEQ ID NOs: 8, 9 and 4, respectively, and light chain variable region CDR1, CDR2 and CDR3 sequences shown in amino acid sequences of SEQ ID NOs: 5, 6 and 7, respectively (the above sequences are obtained according to Chothia numbering system); or heavy chain variable region CDR1, CDR2 and CDR3 sequences shown in amino acid sequences of SEQ ID NOs: 14, 10 and 11, respectively, and light chain variable region CDR1, CDR2 and CDR3 sequences shown in amino acid sequences of SEQ ID NOs: 12, 13 and 7, respectively; or heavy chain variable region CDR1, CDR2 and CDR3 sequences shown in amino acid sequences of SEQ ID NOs: 14, 10 and 11, respectively, and light chain variable region CDR1, CDR2 and CDR3 sequences shown in amino acid sequences of SEQ ID NOs: 12, 15 and 7, respectively.

All the above sequences comprise amino acid sequences that can reach 85%, 87%, 90%, 92%, 93%, 94%, 95%, 96%, 97%, 98% or 99% or more identity.

**A second aspect of the present invention provides variable region sequences of an antibody or antigen-binding fragment thereof capable of binding to canine PD-1.**

The antibody or antigen-binding fragment thereof capable of binding to canine PD-1 comprises a heavy chain variable region selected from SEQ ID NOs: 16 and 18-20 and/or a light chain variable region selected from SEQ ID NOs: 17 and 21-23;
further, the antibody or antigen-binding fragment thereof capable of binding to canine PD-1 may comprise:
a heavy chain variable region shown in SEQ ID NO: 16, and a light chain variable region shown in SEQ ID NO: 17;
or a heavy chain variable region shown in SEQ ID NO: 18, and a light chain variable region shown in SEQ ID NO: 21;
or a heavy chain variable region shown in SEQ ID NO: 18, and a light chain variable region shown in SEQ ID NO: 22;
or a heavy chain variable region shown in SEQ ID NO: 18, and a light chain variable region shown in SEQ ID NO: 23;
or a heavy chain variable region shown in SEQ ID NO: 19, and a light chain variable region shown in SEQ ID NO: 21;
or a heavy chain variable region shown in SEQ ID NO: 19, and a light chain variable region shown in SEQ ID NO: 22;
or a heavy chain variable region shown in SEQ ID NO: 19, and a light chain variable region shown in SEQ ID NO: 23;
or a heavy chain variable region shown in SEQ ID NO: 20, and a light chain variable region shown in SEQ ID NO: 21;
or a heavy chain variable region shown in SEQ ID NO: 20, and a light chain variable region shown in SEQ ID NO: 22;
or a heavy chain variable region shown in SEQ ID NO: 20, and a light chain variable region shown in SEQ ID NO: 23;
or variable regions having amino acid sequences having at least 85%, 87%, 90%, 92%, 93%, 94%, 95%, 96%, 97%, 98% or 99% or more identity to the above sequences.

**A third aspect of the present invention provides constant regions of an antibody or antigen-binding fragment thereof capable of binding to canine PD-1.**

The heavy chain constant region used in the antibody or antigen-binding fragment thereof capable of binding to canine PD-1 may be that of canine IgG A, IgG B, IgG C, IgG D, or a mutant sequence that ensure the same function, and further preferably an IgG B mutant sequence; and a specific amino acid sequence of the heavy chain constant region domain may be shown in SEQ ID NO: 24. The light chain constant region used in the antibody may be a constant region domain sequence of a canine kappa chain or lambda chain, and further preferably a canine kappa chain; and the amino acid sequence is shown in SEQ ID NO: 25.

However, the inventive concept of the present invention is not limited to the constant regions shown in the above exemplary sequences, the constant region designs that can achieve the prevention, treatment, detection and other effects based on the PD-1 target according to the present invention are all within the scope of protection of the present invention.

**A fourth aspect of the present invention provides full-length sequences of an antibody or antigen-binding fragment thereof capable of binding to canine PD-1.**

The antibody or antigen-binding fragment thereof capable of binding to canine PD-1 comprises a full length of a heavy chain and/or a full length of a light chain, the sequence of the full length of the heavy chain used is selected from any of SEQ ID NOs: 26 and 28-30, and the full length of the light chain used in the antibody or antigen-binding fragment capable of binding to canine PD-1 is selected from any of SEQ ID NOs: 27 and 31-33;
the affinity constant KD between the antibody or antigen-binding fragment thereof capable of binding to canine PD-1 and canine PD-1 is in the range of 8.76E-10 to 3.83E-09 M;
further, in the antibody or antigen-binding fragment capable of binding to canine PD-1, the full-length of the heavy chain is shown in SEQ ID NO: 26, and the full-length of the light chain is shown in SEQ ID NO: 27;
or the full length of the heavy chain is shown in SEQ ID NO: 28, and the full length of the light chain is shown in SEQ ID NO: 31;
or the full length of the heavy chain is shown in SEQ ID NO: 28, and the full length of the light chain is shown in SEQ ID NO: 32;
or the full length of the heavy chain is shown in SEQ ID NO: 28, and the full length of the light chain is shown in SEQ ID NO: 33;
or the full length of the heavy chain is shown in SEQ ID NO: 29, and the full length of the light chain is shown in SEQ ID NO: 31;
or the full length of the heavy chain is shown in SEQ ID NO: 29, and the full length of the light chain is shown in SEQ ID NO: 32;
or the full length of the heavy chain is shown in SEQ ID NO: 29, and the full length of the light chain is shown in SEQ ID NO: 33;
or the full length of the heavy chain is shown in SEQ ID NO: 30, and the full length of the light chain is shown in SEQ ID NO: 31;
or the full length of the heavy chain is shown in SEQ ID NO: 30, and the full length of the light chain is shown in SEQ ID NO: 32;
or the full length of the heavy chain is shown in SEQ ID NO: 30, and the full length of the light chain is shown in SEQ ID NO: 33;
or the full-length amino acid sequences having at least 85%, 87%, 90%, 92%, 93%, 94%, 95%, 96%, 97%, 98% or 99% or more identity to the above sequences.

**A fifth aspect of the present invention provides types of an antibody or antigen-binding fragment thereof capable of binding to canine PD-1.**

The antibody or antigen-binding fragment thereof capable of binding to canine PD-1 comprises at least one type selected from a monoclonal antibody, a polyclonal antibody, a multimeric antibody and a CDR-grafted antibody;
optionally, the antibody or antigen-binding fragment capable of binding to canine PD-1 comprises at least one type selected from a single chain antibody, an Fab antibody, an Fv antibody, a single chain antibody, a single domain antibody, and a minimal recognition unit; and in the case of an antigen-binding fragment, the number of CDR sequences is not limited to 6, and may be less than or more than 6, as long as the antigen-binding fragment can bind to a target antigen based on affinity.

Optionally, the antibody or antigen-binding fragment capable of binding to canine PD-1 comprises at least one of an Fab fragment, an (Fab)₂ fragment, an scFv-Fc fusion protein, an scFv-Fv fusion protein, an Fv fragment, and a minimal recognition unit.

**A sixth aspect of the present invention provides a nucleic acid element.**

The element may be a nucleic acid molecule encoding the aforementioned antibody or antigen-binding fragment thereof capable of binding to canine PD-1. The nucleic acid molecule includes, but is not limited to, a DNA and an RNA; preferably, the nucleic acid molecule is a DNA. However, the scope of protection of the present invention comprises all the nucleic acids and nucleotide sequences that can be expressed as the aforementioned amino acid sequences, including nucleic acid sequences deduced based on various different codon preferences, and those skilled in the art can deduce other nucleotide sequences that can be expressed as the aforementioned amino acid sequences, using conventional techniques in the art. The following sequences are only examples.
Specifically, a heavy chain variable region sequence of the antibody or antigen-binding fragment thereof capable of binding to canine PD-1 may be a DNA coding sequence shown in SEQ ID NOs: 49 and 51-53, and a light chain variable region sequence of the antibody or antigen-binding fragment thereof capable of binding to canine PD-1 may be a DNA coding sequence shown in SEQ ID NOs: 50 and 54-56.

Further, for the antibody or antigen-binding fragment thereof capable of binding to canine PD-1, a nucleotide sequence of the heavy chain variable region is a DNA coding sequence shown in SEQ ID NO: 49, and a nucleotide sequence of the light chain variable region is a DNA coding sequence shown in SEQ ID NO: 50;
for the antibody or antigen-binding fragment thereof capable of binding to canine PD-1, a nucleotide sequence of the heavy chain variable region is a DNA coding sequence shown in SEQ ID NO: 51, and a nucleotide sequence of the light chain variable region is a DNA coding sequence shown in SEQ ID NO: 54;
for the antibody or antigen-binding fragment thereof capable of binding to canine PD-1, a nucleotide sequence of the heavy chain variable region is a DNA coding sequence shown in SEQ ID NO: 51, and a nucleotide sequence of the light chain variable region is a DNA coding sequence shown in SEQ ID NO: 55;
for the antibody or antigen-binding fragment thereof capable of binding to canine PD-1, a nucleotide sequence of the heavy chain variable region is a DNA coding sequence shown in SEQ ID NO: 51, and a nucleotide sequence of the light chain variable region is a DNA coding sequence shown in SEQ ID NO: 56;
for the antibody or antigen-binding fragment thereof capable of binding to canine PD-1, a nucleotide sequence of the heavy chain variable region is a DNA coding sequence shown in SEQ ID NO: 52, and a nucleotide sequence of the light chain variable region is a DNA coding sequence shown in SEQ ID NO: 54;
for the antibody or antigen-binding fragment thereof capable of binding to canine PD-1, a nucleotide sequence of the heavy chain variable region is a DNA coding sequence shown in SEQ ID NO: 52, and a nucleotide sequence of the light chain variable region is a DNA coding sequence shown in SEQ ID NO: 55;
for the antibody or antigen-binding fragment thereof capable of binding to canine PD-1, a nucleotide sequence of the heavy chain variable region is a DNA coding sequence shown in SEQ ID NO: 52, and a nucleotide sequence of the light chain variable region is a DNA coding sequence shown in SEQ ID NO: 56;
for the antibody or antigen-binding fragment thereof capable of binding to canine PD-1, a nucleotide sequence of the heavy chain variable region is a DNA coding sequence shown in SEQ ID NO: 53, and a nucleotide sequence of the light chain variable region is a DNA coding sequence shown in SEQ ID NO: 54;
for the antibody or antigen-binding fragment thereof capable of binding to canine PD-1, a nucleotide sequence of the heavy chain variable region is a DNA coding sequence shown in SEQ ID NO: 53, and a nucleotide sequence of the light chain variable region is a DNA coding sequence shown in SEQ ID NO: 55;
and for the antibody or antigen-binding fragment thereof capable of binding to canine PD-1, a nucleotide sequence of the heavy chain variable region is a DNA coding sequence shown in SEQ ID NO: 53, and a nucleotide sequence of the light chain variable region is a DNA coding sequence shown in SEQ ID NO: 56.

Specifically, for the antibody or antigen-binding fragment thereof capable of binding to canine PD-1, a heavy chain constant region of the canine IgG B mutant may be encoded by a DNA sequence shown in SEQ ID NO: 57, and a light chain constant region of a canine kappa chain may be encoded by a DNA sequence shown in SEQ ID NO: 58.

Specifically, for the antibody or antigen-binding fragment thereof capable of binding to canine PD-1, a full-length heavy chain may be encoded by a DNA sequence shown in any of SEQ ID NOs: 59 and 61-63, and a full-length light chain may be encoded by a DNA sequence shown in any of SEQ ID NOs: 60 and 64-66.

Specifically, for the antibody or antigen-binding fragment thereof capable of binding to canine PD-1, a full-length heavy chain is encoded by a DNA sequence shown in SEQ ID NO: 59, and a full-length light chain is encoded by a DNA sequence shown in SEQ ID NO: 60;
or a full-length heavy chain is encoded by a DNA sequence shown in SEQ ID NO: 61, and a full-length light chain is encoded by a DNA sequence shown in SEQ ID NO: 64;
or a full-length heavy chain is encoded by a DNA sequence shown in SEQ ID NO: 61, and a full-length light chain is encoded by a DNA sequence shown in SEQ ID NO: 65;
or a full-length heavy chain is encoded by a DNA sequence shown in SEQ ID NO: 61, and a full-length light chain is encoded by a DNA sequence shown in SEQ ID NO: 66;
or a full-length heavy chain is encoded by a DNA sequence shown in SEQ ID NO: 62, and a full-length light chain is encoded by a DNA sequence shown in SEQ ID NO: 64;
or a full-length heavy chain is encoded by a DNA sequence shown in SEQ ID NO: 62, and a full-length light chain is encoded by a DNA sequence shown in SEQ ID NO: 65;
or a full-length heavy chain is encoded by a DNA sequence shown in SEQ ID NO: 62, and a full-length light chain is encoded by a DNA sequence shown in SEQ ID NO: 66;
or a full-length heavy chain is encoded by a DNA sequence shown in SEQ ID NO: 63, and a full-length light chain is encoded by a DNA sequence shown in SEQ ID NO: 64;
or a full-length heavy chain is encoded by a DNA sequence shown in SEQ ID NO: 63, and a full-length light chain is encoded by a DNA sequence shown in SEQ ID NO: 65;
or a full-length heavy chain is encoded by a DNA sequence shown in SEQ ID NO: 63, and a full-length light chain is encoded by a DNA sequence shown in SEQ ID NO: 66.

Specifically, the CDR amino acid sequences shown in SEQ ID NOs: 2-15 can correspond to the DNA coding sequences shown in SEQ ID NOs: 35-48, respectively.

The element may be a recombinant vector, including, but not limited to, a cloning vector, an expression vector, a shuttle vector, a viral vector (e.g., a lentiviral vector, an adeno-associated viral vector, a poxvirus-associated vector, etc.), carrying the above nucleic acid molecule. Preferably, the expression vector is a eukaryotic expression vector or a prokaryotic expression vector. The expression vector is a plasmid expression vector, such as pcDNA3.1 and pcDNA3.4.

The element may be a recombinant cell which carries the above-mentioned nucleic acid molecule, or can express a recombinant vector for expression of the aforementioned antibody or antigen-binding fragment thereof capable of binding to canine PD-1; the recombinant cell is obtained by introducing the aforementioned expression vector into a host cell; and the recombinant cell is preferably a eukaryotic cell, and the recombinant cell is preferably a mammalian cell. The recombinant cells include, but are not limited to, Chinese hamster ovary cells (CHOK1), HEK293 cells, and the like.

**A seventh aspect of the present invention relates to a pharmaceutical composition and use thereof.**

The pharmaceutical composition comprises the aforementioned antibody or antigen-binding fragment thereof capable of binding to canine PD-1, or the aforementioned nucleic acid molecule, or the aforementioned expression vector, or the aforementioned recombinant cell.

Optionally, the pharmaceutical composition further comprises a pharmaceutically acceptable auxiliary material and excipient.

The pharmaceutical composition can be prepared as a drug combination or kit, which comprises the aforementioned antibody or antigen-binding fragment thereof capable of binding to canine PD-1 or the pharmaceutical composition as a first active ingredient; and optionally, a second active ingredient for combined use, wherein the first active ingredient and the second active ingredient can be the same or different, and the second active ingredient can include, but not limited to, palladia, cyclophosphamide, doxorubicin, vincristine, prednisolone, etc.

Provided is use of the anti-canine PD-1 antibody or antigen-binding fragment thereof, the nucleic acid molecule, the expression vector, the recombinant cell, the pharmaceutical composition, the drug combination or kit in preventing and/or treating tumor, or in preparing a medicament, or in preparing a kit for detecting PD-1 protein in a biological matrix, wherein the tumor is a tumor of a mammal, including a tumor of a human, a monkey, murine (rat, mouse), a horse, cattle, sheep/goat, a pig, canine, a cat and the like, further preferably a tumor of canine.

The tumor category includes, but is not limited to, "tumor", but refers to a class of diseases characterized by the development of abnormal cells that proliferate uncontrollably and have the ability to infiltrate and destroy normal body tissues. Exemplary tumors include, but are not limited to, squamous cell carcinoma (SCC); papilloma; fibrous papilloma; intraosseous carcinoma; invasive nasal carcinoma; malignant melanoma (MM); fibrosarcoma (FSA); fibroma; rhabdomyosarcoma; angiosarcoma (HAS); granular cell tumors; mixed mesenchymal sarcoma; neurofibrosarcoma; undifferentiated sarcoma; myxosarcoma; chondrosarcoma (CSA); mesenchymal-like osteochondrosarcoma (MLO); osteosarcoma (OSA); transmissible venereal tumors (TVT); mast cell tumors (MCT); lymphosarcoma (LSA); ameloblastoma; adamantoblast adenoma; calcifying epithelial odontogenic tumors; odontoma; myxofibroma; cementoma; odontogenic fibroma; acanthomatous epulis; ossifying epulis; fibromatous epulis; basal cell tumors; ceruminoma; viral papilloma; salivary gland tumors; nasal lymphoma; ceruminous gland cancer (CGC); benign eosinophilic adenoma (rhabdomyoma); chondroma; osteoma; osteochondroma; tracheocarcinoma; leiomyoma; benign laryngeal tumors (e.g., rhabdomyoma or eosinophilic adenoma); mediastinal tumors (e.g., thymoma and mediastinal lymphoma); malignant chest wall tumors (sarcoma); benign chest wall tumors (osteoma and chondroma); primary lung tumors; secondary lung tumors (metastatic lung tumors); cardiac tumors (e.g., angiosarcoma, mesothelioma, myxoma, and ectopic thyroid carcinoma); esophageal cancer; gastric tumors (e.g., gastric adenocarcinoma and lymphoma); gastric leiomyosarcoma; gastric leiomyoma; gastric extramedullary plasmacytoma; gastrointestinal mast cell tumor; non-lymphoid intestinal tumors; intestinal lymphoma; intestinal adenocarcinoma; extramedullary plasmacytoma; carcinoid; adenocarcinomatous polyp; large intestine adenocarcinoma; colorectal leiomyoma and leiomyosarcoma; perianal adenoma; perianal adenocarcinoma; anal cystadenocarcinoma; liver tumors (e.g., liver sarcoma, liver cancer, or carcinoid); hepatocellular carcinoma (HCC); hepatocellular adenoma; hepatoblastoma; biliary tract cancer; adenocarcinoma of the bile duct; gallbladder tumors; pancreatic adenocarcinoma; breast tumors; malignant breast tumors (breast cancer); malignant mixed tumors; breast sarcoma; exoskeletal osteosarcoma of the breast; uterine tumors; vaginal and vulva tumors; ovarian tumors (e.g., adenocystoma, granulosa tumors); genital cord stromal tumors (e.g., thecoma and luteoma); germ cell tumors (dysgerminoma, teratoma, or teratocarcinoma); testicular tumors (e.g., sertoli cell tumors, seminoma, and stromal cell tumors); histiocytic reticuloendothelioma, transitional cell carcinoma (TCC); prostate tumors; kidney tumors (e.g., renal carcinoma, fibrosarcoma and angiosarcoma); tumors of ureter; bladder tumors; tumors of urethra; cutaneous hemangioma; subungual squamous epithelial carcinoma; basal cell tumors (including basal cell carcinoma, basal cell epithelium carcinoma and basal cell-like tumors); ceruminous gland tumors; intradermal keratoepithelioma (keratoacanthoma); hair follicle tumors (trichoblastoma, trichoepithelioma, and nail bed cell tumors); cutaneous plasma cell tumors; cutaneous lymphoma; sebaceous adenoma; sweat gland tumors; epitheliotropic lymphoma (ELSA); cutaneous histiocytosarcoma; cutaneous neuroendocrine (Merkel cell) tumors; cutaneous smooth muscle tumors; soft tissue or spindle cell sarcoma (STS); lymphangiosarcoma; synovial cell sarcoma; histiocytic sarcoma (HS); osteosarcoma of extremities; osteosarcoma of the axial skeleton; extraskeletal osteosarcoma; superficial osteosarcoma; multilobular osteochondrosarcoma (MLO); multiple cartilaginous exostoses (MCE); lymphocytic leukemia (CLL); non-lymphocytic leukemia (granulocytic leukemia); chronic myelogenous leukemia (CML); acute myelogenous leukemia (AML); splenic hemangiosarcoma (HSA); splenic sarcoma (non-lymphocytic, non-vascular); glioma; meningioma; gangliocytoma (pheochromocytoma and nonchromaffin paraganglioma); ganglioglioma; spinal cord tumors (OSA, CSA, FSA, HSA, hemangioendothelioma, and myeloma); peripheral nerve sheath tumors (PNSTs); neuroepithelioma (ependymoma, medulloepithelioma, nephroblastoma, and spinal cord blastoma); intramedullary spinal tumors; epidural sarcoma; epidural sarcoma; nerve sheath tumors; tumors of the peripheral nervous system (e.g., neuroblastoma, medulloblastoma, and retinal neuroblastoma); eyelid tumors; tarsal plate adenoma; myoblastoma; third eyelid tumors; conjunctival tumors; extrabulbar (corneal and scleral) tumors; orbital (the cavity surrounding the eyeball) and retrobulbar tumors; thyroid tumors; parathyroid tumors; APUD cell tumors (amine precursor uptake and decarboxylation cell tumors); insulinoma; glucagonoma; gastrinoma; adrenocortical tumors; pheochromocytoma; and pituitary tumors. Melanoma, mast cell tumor, colon cancer are preferred.

Compared with the prior art, the present invention has the following advantages and beneficial effects:
1. The anti-PD-1 chimeric antibody and the caninized antibodies described in the present invention have good affinity, wherein the affinity constant KD of the chimeric antibody is 8.76E-10 M, and the affinity constant KD of a variety of caninized antibodies is less than 3.83E-09 M.
2. The anti-PD-1 chimeric antibody and the caninized antibodies described in the present invention can effectively block the inhibitory pathway of canine T cell activation (cPD-1/cPD-L1 pathway) in vitro, thereby enhancing T cell activation, and these antibodies have biological activity.
3. The anti-PD-1 chimeric antibody and the caninized antibodies described in the present invention can inhibit MC38 tumor growth in mice at an in vivo level, and also have good anti-tumor efficacy on canine tumors, which is of great significance for the development of highly effective medicaments for cancer treatment.

### Brief Description of the Drawings

The accompanying drawings described herein are used to provide further understanding of the embodiments of the present invention, constitute a part of the present application, and do not constitute a limitation to the embodiments of the present invention. In the drawings:
FIG. 1 shows result diagrams of flow cytometry detection of competitive binding of 3334 chimeric antibody to canine PD-1 with PD-L1 (A is a flow cytometry result diagram; B is a quantitative graph of APC positive cell rates).
FIG. 2 shows result diagrams of in vitro activity detection of 3334 chimeric antibody and caninized antibodies by a luciferase reporter gene method (panel A shows the 3334 chimeric antibody and 3334-H1K1/H1K2/H1K3-canIgG B caninized antibody group; panel B shows the 3334 chimeric antibody and 3334-H2K1/H2K2/H2K3-canIgG B caninized antibody group; and panel C shows the 3334 chimeric antibody and 3334-H3K1/H3K2/H3K3-canIgG B caninized antibody group).
FIG. 3 shows a result diagram of in vitro activity detection of 3334 chimeric antibody and caninized antibodies by the T cell activation experiment.
FIG. 4 shows a result diagram of the effect of 3334 chimeric antibody and 3334-H1K2-canIgG B caninized antibody on the body weight of mice.
FIG. 5 shows a result diagram of the effect of 3334 chimeric antibody and 3334-H1K2-canIgG B caninized antibody on the body weight change rate of mice.
FIG. 6 shows a result diagram of the effect of 3334 chimeric antibody and 3334-H1K2-canIgG B caninized antibody on the tumor volume of mice.

### Detailed Description of Embodiments

The following examples serve to illustrate the present invention and are not intended to limit the scope of the present invention.

The experimental methods used in the examples below are all conventional methods, unless otherwise specified.

All the materials, reagents, etc., used in the following examples can be obtained from commercial sources, unless otherwise specified.

The present invention will be described in detail below by way of examples in conjunction with the accompanying drawings, which are not intended to limit the present invention and are merely illustrative.

In order to understand the present disclosure more clearly, some terms are firstly defined. The terms "first" and "second" are only used for illustrative purposes, and can not be understood as indicating or implying relative importance or implicitly indicating the number of the indicated technical feature. Thus, the features defined with "first" and "second" may explicitly or implicitly comprise one or more of the features. Further, in the description of the present invention, unless otherwise specified, "a plurality of" means two or more.

The term "comprising" or "containing" herein is an open-ended expression, i.e., including the contents specified in the present invention, but not excluding contents in other aspects.

The terms "optionally" or "optional" herein generally mean that the subsequently described event or condition may, but is not necessary to, occur, and that the description comprises instances in which the event or condition occurs, as well as instances in which the event or condition does not occur.

The term "antibody" herein generally refers to an antibody capable of recognizing one or more antigenic epitopes, including but not limited to a monoclonal antibody, a polyclonal antibody, a multimeric antibody and a CDR-grafted antibody, which is an immunoglobulin molecule capable of binding to a specific antigen. The antibody comprises two light chains with lighter molecular weights and two heavy chains with heavier molecular weights. The heavy chains (H chains) and light chains (L chains) are linked by disulfide bonds to form a molecule of four peptide chains. In the molecule, the amino-terminal (N-terminal) amino acid sequences of the peptide chains change greatly, which are called variable regions (V regions), and the carboxyl-terminal (C-terminal) amino acid sequences are relatively stable and change little, which are called constant regions (C regions). The V regions of the L chain and H chain are referred to as VL and VH, respectively. In the variable regions, the amino acid composition and arrangement sequence of some regions have a higher degree of change, which are called hypervariable regions (hypervariable regions, HVRs). The hypervariable regions constitute the position where an antigen binds to the antibody, and thus they are also called complementarity-determining regions (CDRs). There are three CDRs on both the heavy chain variable region and the light chain variable region.

The term "Fab antibody" herein generally refers to an antibody containing only an Fab molecule, which is composed of the VH and CH1 of a heavy chain and a complete light chain, wherein the light chain and the heavy chain are linked by one disulfide bond.

The term "Fv antibody" herein generally refers to an antibody formed by linking only a light chain variable region (VL) and a heavy chain variable region (VH) by a non-covalent bond, which is the smallest functional fragment of an antibody that retains an intact antigen-binding site.

The terms "single domain antibody", "nanobody" and "VHH antibody" herein are used interchangeably, originally described as an antigen-binding immunoglobulin (variable) domain of a "heavy chain antibody" (i.e. "an antibody lacking light chains") (Hamers-Casterman C, AtarhouchT, Muyldermans S, Robinson G, Hamers C, Songa EB, Bendahman N, Hamers R.: "Naturallyoccurring antibodies devoid of light chains"; Nature 363, 446-448(1993)), which comprises only a heavy chain variable region (VH) and conventional CH2 and CH3 regions, and specifically binds to an antigen via the heavy chain variable region.

The term "single-chain antibody" herein generally refers to an antibody in which a heavy chain variable region (VH) and a light chain variable region (VL) are linked via a linker peptide.

The terms "minimal recognition unit" and "MRU" herein both refer to an antibody consisting of only one CDR, which has a very small molecular weight accounting for only about 1% of that of a complete antibody.

The term "identity", "homology" or "similarity" used herein means when an amino acid or nucleic acid sequence is described with respect to a reference sequence, the percentage of identical amino acids or nucleotides between the two amino acid sequences or nucleic acid sequences determined by conventional methods. For example, reference can be made to Ausubel et al., ed. (1995), Current Protocols in Molecular Biology, Chapter 19 (Greene Publishing and Wiley-Interscience, New York); and ALIGN program (Dayhoff (1978), Atlas of Protein Sequence and Structure 5: Suppl.3 (National Biomedical Research Foundation, Washington, D.C.). There are many algorithms for aligning sequences and determining sequence identity, including the homology alignment algorithm described in Needleman et al. (1970) J. Mol. Biol. 48:443; the local homology algorithm described in Smith et al. (1981) Adv. Appl. Math. 2:482; the similarity search method described in Pearson et al. (1988) Proc. Natl. Acad. Sci. 85:2444; the Smith-Waterman algorithm (Meth. Mol. Biol. 70:173-187(1997); and BLASTP, BLASTN, and BLASTX algorithms (see Altschul et al. (1990) J. Mol. Biol. 215:403-410). Computer programs that utilize these algorithms are also available and include, but are not limited to: ALIGN or Megalign (DNASTAR) software, or WU-BLAST-2 (Altschul et al., Meth. Enzym., 266:460-480(1996)); or GAP, BESTFIT, BLAST (Altschul et al., supra), FASTA, and TFASTA, which can be available in the Genetics Computing Group (GCG) package, version 8, Madison, Wisconsin, USA; and CLUSTAL in the PC/Gene program provided by Intelligenetics, Mountain View, California.

The term "at least 85% identity" herein refers to at least 85% identity, that may be 85%, 86%, 87%, 88%, 89%, 90%, 91%, 92%, 93%, 94%, 95%, 96%, 97%, 98%, 99%, 99.5% and 99.9% identity, to the corresponding reference sequence.

As used herein, an antibody that "binds to PD-1" or "specifically binds to PD-1", or an "anti-PD-1 antibody" refers to an antibody that binds to the PD-1 with a KD value of 1.0 × 10⁻⁸ mol/L or less. Binding affinities are determined using PD-1 extracellular domains by standard binding assays, such as bio-layer interferometry (Gator).

The term "expression vector" herein generally refers to a nucleic acid molecule which can be inserted into a suitable host to self-replicate, which transfers an inserted nucleic acid molecule into a host cell and/or between host cells. The expression vector may comprise a vector mainly for inserting a DNA or RNA into a cell, a vector mainly for replicating a DNA or RNA, and a vector mainly for expression of transcription and/or translation of a DNA or RNA. The expression vector also includes a vector having a variety of the above-mentioned functions. The expression vector may be a polynucleotide which can be transcribed and translated into a polypeptide, when introduced into a suitable host cell. Typically, the expression vector can produce a desired expression product by culturing a suitable host cell comprising the expression vector. The expression vector carries the aforementioned nucleic acid molecule. When the above-mentioned nucleic acid molecule is linked to the vector, the nucleic acid molecule may be directly or indirectly linked to control elements on the vector, as long as these control elements can control the translation, expression and the like of the nucleic acid molecule. Of course, these control elements may be derived directly from the vector itself, or may be exogenous, i.e., not derived from the vector itself. Of course, the nucleic acid molecule may be operably linked to a control element. The expression "operably linked" herein means that a foreign gene is linked to the vector, so that the control element within the vector, such as a transcription control sequence, a translation control sequence, etc., can exert its intended function of regulating transcription and translation of the foreign gene. Commonly used vectors may be, for example, plasmids, phages, and the like. After the expression vector according to some specific embodiments of the present invention is introduced into a suitable recipient cell, the expression of the aforementioned antibody or antigen-binding fragment can be effectively achieved under the mediation of a regulatory system, and then the antibody or antigen-binding fragment can be obtained in large quantities in vitro.

The term "recombinant cell" herein generally refers to a cell having a unique trait stably inherited, which is obtained by performing modifying or recombination on the genetic material of a host cell using a genetic engineering technique or a cell fusion technique. The term "host cell" herein refers to a prokaryotic or eukaryotic cell into which a recombinant expression vector can be introduced. As used herein, the term "transformation" or "transfection" refers to the introduction of a nucleic acid (e. g., a vector) into a cell by various techniques known in the art. A suitable host cell may be transformed or transfected with the DNA sequence of the present invention and may be used for expression and/or secretion of the target protein.

The term "pharmaceutical composition" herein generally refers to a unit dosage form and may be prepared by any of the methods well known in the pharmaceutical art. All the methods comprise the step of bringing an active ingredient into association with carriers which constitutes one or more accessory ingredients. In general, the composition is prepared by uniformly and thoroughly bringing an active compound into association with a liquid carrier, a finely divided solid carrier or both.

The term "pharmaceutically acceptable auxiliary material" herein may include any solvent, solid excipient, diluent, or other liquid excipients, etc., suitable for the particular intended dosage form. Its use is also within the scope considered by the present invention, except in cases where any conventional auxiliary material is incompatible with the compound of the present invention, for example, any adverse biological effects produced or any interaction occurring in a harmful manner with any other component of the pharmaceutically acceptable composition.

The term "administering" herein refers to introducing a predetermined amount of a substance into a treatment subject through some suitable means. The antibody or antigen-binding fragment or pharmaceutical composition of the present invention may be administered by any common route, as long as it can reach the intended tissue. Various modes of administration are contemplated, including intraperitoneal injection, intravenous injection, intravenous drip, intramuscular injection, subcutaneous injection, and the like; however, the present invention is not limited to these exemplified modes of administration. The treatment subject may be a mammal, preferably canine.

The term "treatment" herein refers to obtaining a desired pharmacological and/or physiological effect. The effect may be prophylactic in terms of completely or partially preventing a disease or symptoms thereof, and/or may be therapeutic in terms of partially or completely curing a disease and/or an adverse effect resulting from a disease. As used herein, "treatment" encompasses that for a disease in a mammal, particularly canine, and comprises: (a) preventing the disease or condition from occurring in an individual who is susceptible to the disease but has not yet been diagnosed with the disease; (b) inhibiting the disease, e.g., blocking the progression of the disease; or (c) relieving the disease, e.g., alleviating the symptoms associated with the disease. As used herein, "treatment" encompasses any medication in which a medicament or compound is administered to an individual to treat, cure, relieve, ameliorate, alleviate or inhibit a disease in the individual, including, but not limited to, administering a medicament comprising the compound described herein to an individual in need thereof.

### Example 1 Preparation and identification of a mouse anti-canine PD-1 antibody

### 1. Preparation of the antigen

The UniProt database and GenBank database were searched for canine PD-1 protein (UniProtKB: A0A024FCJ9), the amino acid sequence thereof was shown in SEQ ID NO: 1, the corresponding nucleotide sequence with a His tag was artificially synthesized and constructed in pcDNA3.4 vector. The vector was transiently transfected into HEK293 cells, the cell supernatant was collected 5 days after transfection, the supernatant was subjected to an affinity chromatography by Ni column and the purified canine PD-1 recombinant protein was obtained by elution.

### 2. Immunization of mice

Balb/c mice or C57 mice were immunized with 7-8 mg canine PD-1 recombinant protein as the antigen. Sera were collected after four immunizations and analyzed for antibody titer using enzyme-linked immunosorbent assay (ELISA).

### 3. Spleen RNA extraction and reverse transcription

RNAs was extracted from a mouse spleen tissue by Trizol method, the integrity of RNAs was identified by denaturing agarose gel electrophoresis, and the concentration of RNAs and OD₂₆₀/OD₂₈₀ were measured by an ultramicro spectrophotometer. Qualified RNAs were selected, and RNA reverse transcription was performed according to the instruction of Superscript II reverse transcriptase (Thermo Scientific) to obtain cDNAs.

### 4. Construction of an antibody library and phage display

The antibody heavy chain and light chain were amplified from cDNA, the scFv antibody library and Fab antibody library were constructed respectively, with the library capacity of 1×10⁸ CFU/mL. The antibody library was transferred into TG1 E. coli through electroporation, then the helper phage M13KO7 was added to infect TG1 E. coli, and after overnight culture, the phage was precipitated using PEG/NaCl and a phage display antibody library was prepared.

### 5. Screening for phage display antibody libraries

Solid phase screening and solid phase and liquid phase cross-screening for the phage display antibody library were performed by an immune tube and a magnetic bead screening instrument. In the solid phase screening, the canine PD-1 recombinant protein was used to coat the immune tube, and in the liquid phase screening, biotinylated canine PD-1 recombinant protein was used to coat Dynabeads magnetic beads. After three rounds of screening, Phage ELISA was used to identify the enriched phage collection as well as the single clone isolated from the collection.

### 6. Construction, expression and purification of the antibody

The phagemid of Phage ELISA-positive single clone was subjected to a DNA sequencing, and based on the sequencing results, the murine antibody variable region with a correct reading frame was selected for gene synthesis to obtain an active murine antibody. Amino acid sequences of complementarity determining regions (CDRs) 1, 2 and 3 of heavy and light chain variable regions of murine antibodies were obtained using Kabat, Chothia, and IMGT numbering (numbered in the abYsis and IMGT databases, respectively) respectively.

Then, the heavy chain variable region (SEQ ID NO: 16) of murine antibody was fused with the heavy chain constant region (including CH1, CH2 and CH3) from a canine IgG B mutant, the light chain variable region (SEQ ID NO: 17) was fused with the canine kappa chain constant region (CL), and they were inserted into pcDNA3.4 vector to construct a full-length chimeric antibody expression plasmid. The chimeric antibody was transiently transfected and expressed in HEK293 cells, the culture supernatant was subjected to recombinant protein A affinity chromatography to obtain a purified chimeric antibody, numbered 3334, wherein the full length of the heavy chain of the chimeric antibody was shown in SEQ ID NO: 26, and the full length of the light chain was shown in SEQ ID NO: 27.

### Example 2 Screening for antibodies that block the binding of canine PD-1 to its ligand

The full-length canine PD-L1 (UniProtKB: E2RKZ5) was constructed and transfected into CHO-K1 cells, and a monoclonal cell line with high expression (hereinafter referred to as canine PD-L1/CHO-K1 cells for short) was obtained by screening. The ability of anti-canine PD-1 antibodies to block the binding of canine PD-1 to canine PD-L1/CHO-K1 cells was detected by flow cytometry.

Biotin-labelled canine PD-1 recombinant protein was incubated with different concentrations of antibodies for 5 minutes, then added to PD-L1/CHO-K1 cells, incubated at 4°C for 60 minutes, and rinsed twice with cold PBS containing 1% FBS. Streptavidin-APC fluorescent secondary antibody was added. The mixture was incubated at 4°C for 30 minutes, and rinsed twice with cold PBS containing 1% FBS, and resuspended in 200 mL PBS containing 1% FBS, and the proportion of cells labelled by APC was analyzed using a flow cytometer (Beckman Coulter, CytoFLEX). The APC positive cell rate reflects the binding ability of the biotin-labelled canine PD-1 recombinant protein to PD-L1/CHO-K1 cells. The lower the APC positive cell rate, the stronger the ability of the antibodies to block the binding of biotin-labelled canine PD-1 recombinant protein to its ligand.

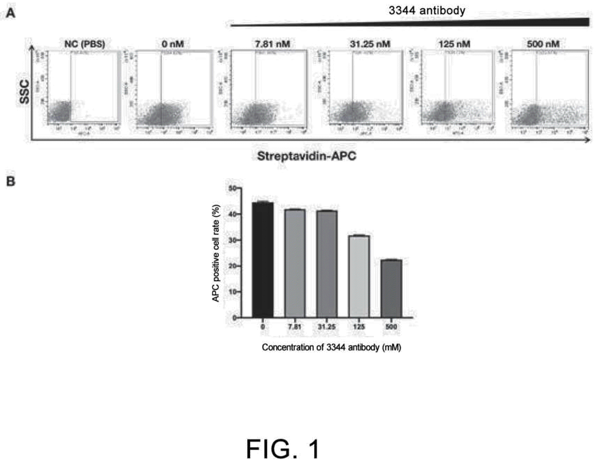

As shown in the result of FIG. 1, with the increase of the concentration of 3334 chimeric antibody, the APC positive cell rate gradually decreased, indicating that 3334 chimeric antibody can block the binding of canine PD-1 to PD-L1.

### Example 3 Construction of caninized antibodies

The 3334 chimeric antibody was caninized by CDR grafting and back mutation based on framework region homology. Firstly, caninized heavy chain variable region variants (SEQ ID NOs: 18-20) and caninized light chain variable region variants (SEQ ID NOs: 21-23) were constructed. A variable region of a caninized antibody was obtained by freely combining with a caninized heavy chain variable region and a caninized light chain variable region of the same antibody. For example, heavy chain 3334-H1 (SEQ ID NO: 18) can be combined with light chain 3334-K1 (SEQ ID NO: 21), 3334-K2 (SEQ ID NO: 22) or 3334-K3 (SEQ ID NO: 23), and the combinations were labelled as 3334-H1K1, 3334-H1K2 and 3334-H1K3 respectively. A constant region of a caninized antibody consisted of a constant domain of a canine IgG B mutant and a canine kappa constant region domain. For example, 3334-H1K1-canIgG B was composed of a caninized variable region heavy chain H1, a caninized variable region light chain K1, a constant region of a canine IgG B mutant and a canine kappa constant region. For the details of specific sequence combination names, reference can be made to Table 1. The constant regions of the antibodies shown were a heavy chain constant region domain of a canine IgG B mutant, with an amino acid sequence shown in SEQ ID NO: 24, and a canine kappa chain constant region domain with an amino acid sequence shown in SEQ ID NO: 25, respectively.

**Table 1 Sequence numbering list of 3334 chimeric antibody and caninized antibodies**

| Type | Antibody name |
|---|---|
| Chimeric antibody | 3334-canIgG B |
| 3334-based caninized antibodies | 3334-H1K1-canIgG B |
| | 3334-H1K2-canIgG B |
| | 3334-H1K3-canIgG B |
| | 3334-H2K1-canIgG B |
| | 3334-H2K2-canIgG B |
| | 3334-H2K3-canIgG B |
| | 3334-H3K1-canIgG B |
| | 3334-H3K2-canIgG B |
| | 3334-H3K3-canIgG B |

### Example 4 Affinity assay of the constructed chimeric antibody and caninized antibodies

Affinities of 3334 chimeric antibody and nine 3334 caninized antibodies were determined using a Gator instrument based on the Bio-Layer Interferometry (BLI) technology. Firstly, the antibodies were labelled with biotin (ThermoFisher, 21343), and the biotinylated test antibodies were diluted to 100 nM and added to a black 96-well plate as the stationary phase. Then the analyte (canine PD-1-His) was diluted in gradient (200, 100, 50, 25, 12.5, 6.25, 3.13 nM) and added to the black 96-well plate. Finally, streptavidin probes (Gator^{™} Streptavidin Probes) were used to determine the affinities of the antibodies to the canine PD-1 according to the movement procedure of equilibration, stationary phase, equilibration, analyte, and equilibration.

The results were shown in Table 2, the affinity of the 3334 chimeric antibody to PD-1 was 8.76E-10, and the affinities of the 3334 caninized antibodies were slightly weaker than that of the 3334 chimeric antibody, but the change was not significant.

**Table 2 Affinity data of 3334 chimeric antibody and 3334 caninized antibodies**

| Anti-canine PD-1 antibody | Dissociation rate constant, Koff (1/s) | Association rate constant, Kon (1/Ms) | Affinity KD (M) |
|---|---|---|---|
| 3334-canIgG B | 2.59E-04 | 2.96E+05 | 8.76E-10 |
| 3334-H1K1-canIgG B | 4.44E-04 | 2.42E+05 | 1.84E-09 |
| 3334-H1K2-canIgG B | 2.74E-04 | 1.94E+05 | 1.41E-09 |
| 3334-H1K3-canIgG B | 3.31E-04 | 1.65E+05 | 2.01E-09 |
| 3334-H2K1-canIgG B | 4.57E-04 | 1.33E+05 | 3.45E-09 |
| 3334-H2K2-canIgG B | 8.00E-04 | 2.46E+05 | 3.25E-09 |
| 3334-H2K3-canIgG B | 4.15E-04 | 1.25E+05 | 3.33E-09 |
| 3334-H3K1-canIgG B | 1.30E-03 | 3.40E+05 | 3.83E-09 |
| 3334-H3K2-canIgG B | 5.94E-04 | 1.95E+05 | 3.04E-09 |
| 3334-H3K3-canIgG B | 9.43E-04 | 2.74E+05 | 3.44E-09 |

| | | | |
|---|---|---|---|
| Notes: E is a scientific notation, which is × 10, and the value after E is the exponent of 10. | | | |

### Example 5 In vitro activity assay of the chimeric antibody and caninized antibodies

### 1. Detection of in vitro biological activity of new antibodies using canine PD-1/PD-L1 luciferase reporter gene-containing cell lines

The canine PD-1/PD-L1 luciferase reporter gene-containing monoclonal cell lines of Canine_PD-L1 aAPC CHO-K1 and Canine_PD-1 Reporter Jurkat based on the canine PD-1/PD-L1 signalling pathway, were constructed respectively, for evaluating the ability of antibodies to affect PD-1/PD-L1 signalling pathway. The test antibodies were 3334 chimeric antibody and 9 caninized antibodies.

Firstly, the Canine_PD-L1 aAPC CHO-K1 cell line as the target cell was plated in a 96-well cell plate at a density of 1E4 cells/well 16-24 h in advance. Then, 50 µL of the Canine_PD-1 Reporter Jurkat cell line as the effector cell at 2E6 cells/mL was incubated with 50 µL of 3334 chimeric antibody and caninized antibodies at serial concentrations for 1 h, respectively. After that, the culture media of the target cells in the 96-well cell plate were removed, the mixed solution of the above effector cells and antibodies was added, and the incubation was continued for 16 h. Finally, the suspension cells in the supernatant were taken, and 50 µL of ONE-Glo enzyme reaction substrate (Promega, E6120) was added. After incubation at room temperature in dark for 15 min, the fluorescence signal was detected on machine. The EC₅₀ was calculated using Prism software.

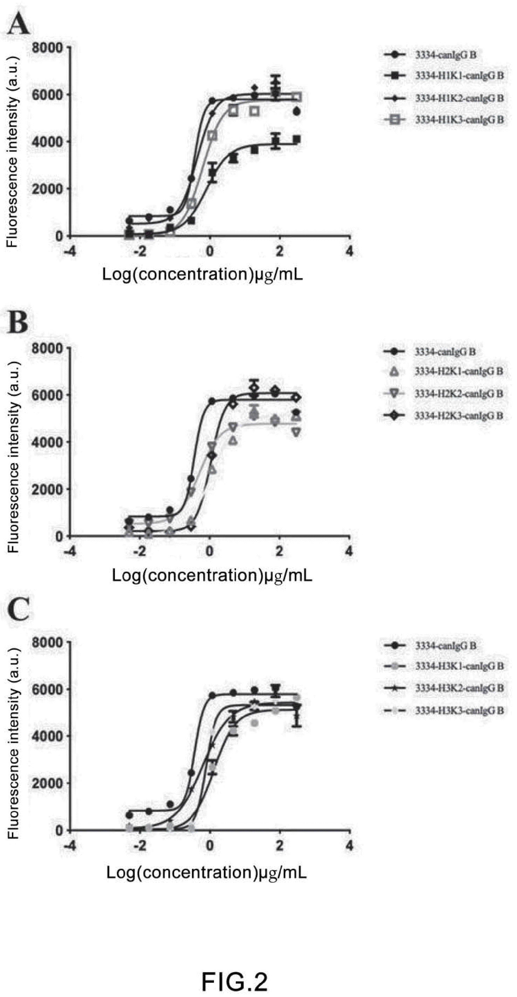

The results were shown in Table 3 and FIG. 2, indicating that 3334 chimeric antibody and 9 caninized antibodies can effectively block the PD-1/PD-L1 signalling pathway, and the EC₅₀ value was 0.36-1.23 µg/mL.

**Table 3 In vitro cell biological activity data of 3334 chimeric antibody and caninized antibodies**

| **Anti-canine PD-1 antibody** | **EC₅₀ (µg/mL)** |
|---|---|
| 3334-canIgG B | 0.36 |
| 3334-H1K1-canIgG B | 0.72 |
| 3334-H1K2-canIgG B | 0.37 |
| 3334-H1K3-canIgG B | 0.59 |
| 3334-H2K1-canIgG B | 1.07 |
| 3334-H2K2-canIgG B | 0.42 |
| 3334-H2K3-canIgG B | 1.06 |
| 3334-H3K1-canIgG B | 1.23 |
| 3334-H3K2-canIgG B | 0.55 |
| 3334-H3K3-canIgG B | 0.75 |

### 2. Further detection of in vitro biological activity of 3334 chimeric antibody and caninized antibodies using a T cell activation experiment

Healthy adult Beagle dogs were selected to collect whole blood, and canine PBMCs were extracted by Ficoll (GE Healthcare, 17-1440-02) density gradient centrifugation. Canine PBMCs were added to a 96-well cell plate at a density of 4E5 cells/well, and 10 ng/mL staphylococcal enterotoxin B from Staphylococcus aureus (SEB, Toxin Technology, BT202) was added as a superantigen for stimulation. Simultaneously, negative control (canIgG B), 3334 chimeric antibody and caninized antibodies were added respectively, and incubated in a carbon dioxide incubator at 37°C for 72 h, and the cell supernatant was collected by centrifugation. The contents of cIFN-γ (R&D, DY781B) and cIL-2 (R&D, DY1815) in the supernatant were detected using an ELISA assay kit.

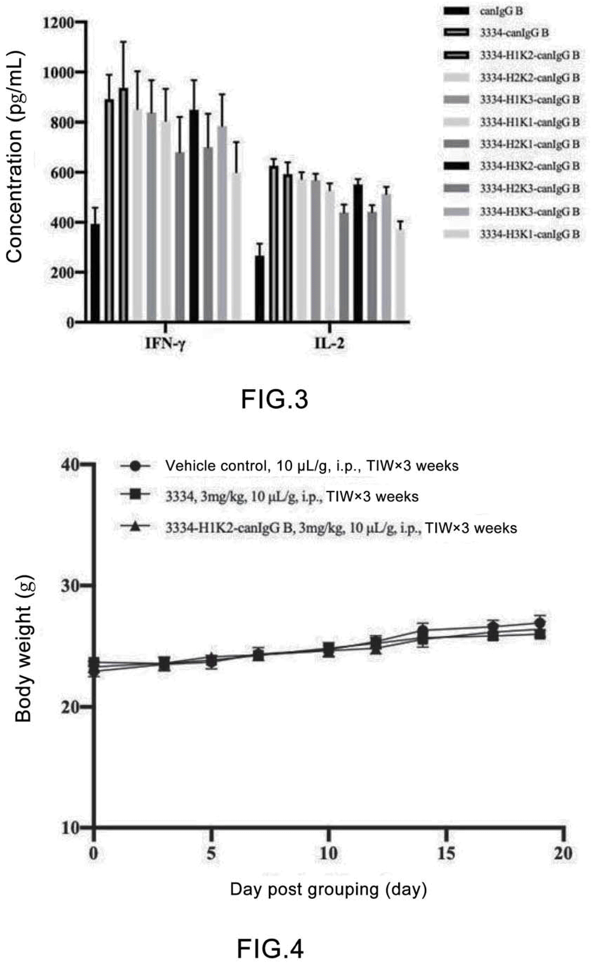

The results were shown in FIG. 3, the 3334 chimeric antibody and 9 caninized antibodies can increase the secretion of T cell cIFN-γ and cIL-2 at an in vitro level, and the difference was significant compared with the negative control. These results indicate that the 3334 antibodies can effectively block the inhibitory pathway of canine T cell activation (cPD-1/cPD-L1 pathway) in vitro, thereby enhancing T cell activation, and these antibodies have biological activity.

### Example 6 In vivo efficacy evaluation of 3334 chimeric antibody and 3334-H1K2-canIgG B antibody

Previous validation showed that canine PD-1 can cross-bind to human PD-L1 (hPD-L1). Therefore, the colon cancer MC38/hPD-L1 recombinant cell line was subcutaneously transplanted to female canine PD-1 C57BL/6 transgenic mice for establishing an animal model for detection, to further evaluate the anti-tumor effect of 3334 chimeric antibody and 3334-H1K2-canIgG B.

MC38/hPD-L1 cells at the exponential growth phase were collected, and resuspended to 1E7/mL in PBS buffer, and 0.1 mL of MC38/hPD-L1 cells were subcutaneously inoculated on the right back of canine PD-1 C57BL/6 transgenic mice, respectively. All animals were weighed and tumor volumes were measured with a vernier caliper, and when tumors grew to an average volume of 102.10 mm³, the animals were randomized according to tumor size and dosed. The experimental groups were negative control group (vehicle control), 3334 chimeric antibody group and 3334-H1K2-canIgG B antibody group, with 6 mice in each group. On the day of grouping (day 0), the drug administration started, i.e. the corresponding antibodies were injected at 3 mg/kg via the tail vein of the mice in the antibody groups, and an equal volume of vehicle was injected via the tail vein of the mice in the negative control group. The administration frequency was 3 times a week for a total of 3 weeks. After the administration, the activities, food intake and water consumption, weight gain and loss, eyes, coat and some abnormal conditions of the experimental animals were observed every day, and the body weight and tumor size of the mice were measured three times a week. Tumor volume calculation formula: tumor volume (mm³) = 1/2 × (a × b²) (wherein a represents the long diameter and b represents the short diameter). The experiment was finished after 9 times of administration.

The anti-tumor effect of 3334 antibodies was evaluated using absolute tumor proliferation rate (T/C%) and absolute tumor growth inhibition rate (TGI%). T/C% = Ti/Ci × 100%, TGI% = (Ci-Ti) / Ci×100%, wherein Ti represents the average tumor volume of the administration group on day i, and Ci represents the average tumor volume of the negative control group on day i.

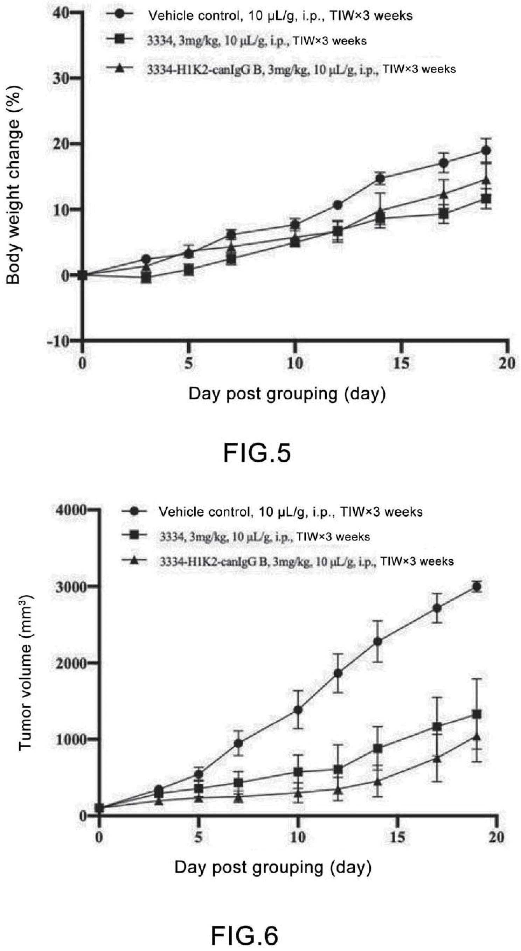

The results were shown in FIGS. 4-6. FIGS. 4 and 5 showed that the body weight of mice in each group increased during the administration period, and there was no significant difference in body weight changes between groups. Furthermore, there was no unexpected death of mice during the treatment, indicating that each administration group had good tolerance without significant toxic reactions. FIG. 6 showed the tumor growth of each group, in which the tumor growth rates of mice in the 3334 antibody group and the 3334-H1K2-canIgG B antibody group reduced significantly, as compared with the negative control group.

The statistical results of T/C% and TGI% after the last administration were shown in Table 4. Mice in both the 3334 chimeric antibody group and the 3334-H1K2-canIgG B antibody group survived on day 19, with mean tumor volumes of 1333.08 mm³ and 1044.91 mm³, respectively, and TGI of 55.54 and 65.15, respectively. There were statistically significant and extremely significant differences compared with the negative control group, respectively.

**Table 4 Data of inhibition of MC38 tumor growth in mice by 3334 chimeric antibody and 3334-H1K2-canIgG B antibodies**

| Gro up | Dosing | Dose (mg/k g) | Number of mice at day 0/day 7 | Day 19 | | | |
|---|---|---|---|---|---|---|---|
| | | | | Mean tumor volume (mm³) ± SEM | T/C (%) | TGI (%) | P Value |
| 1 | Vehicle control | - | 6/5 | 2998.66±70.14 | - | - | - |
| 2 | 3334-canIg G B | 3 | 6/6 | 1333.08±462.06 | 44.46 | 55.54 | <0.05* |
| 3 | 3334-H1K2-canIgG B | 3 | 6/6 | 1044.91±338.25 | 34.85 | 65.15 | <0.01** |

### Example 7 In vivo efficacy evaluation of 3334 chimeric antibody and 3334-H1K2-canIgG B antibody in target animals

This example serves to illustrate the therapeutic effect of 3334 and 3334-H1K2-canIgG B on canine malignant tumors.

A single-arm trial design was used. Animals were screened from a pet hospital in Beijing and 40 tumor-affected dogs seeking medical treatment (including 20 with melanoma and 20 with mast cell tumors) were enrolled. The efficacy of 3334 chimeric antibody and 3334-H1K2-canIgG B antibody in the treatment of malignant tumor-affected dogs was studied, respectively. The varieties of experimental dogs included: Schnauzer, Labrador, Chihuahua, Golden Retriever, etc., with an average age of 12 years old (10-15 years old), all of which were dogs with advanced malignant tumors.

All diseased dogs were divided into two groups, 3334 chimeric antibody group and 3334-H1K2-canIgG B group, each group included 10 dogs with melanoma and 10 dogs with mast cell tumors. For all diseased dogs, the corresponding 3334 chimeric antibody and 3334-H1K2-canIgG B antibody (diluted with normal saline for injection) were intravenously dripped at a dose of 5 mg/kg body weight, the administered was performed once every two weeks, for 8 consecutive times, and the trial was finished one week after the last administration. Tumor burden was assessed by gross examination and computed tomography, the tumor size was measured every two weeks to assess efficacy, and objective response rate (ORR), complete response (CR) rate and partial response (PR) rate were compared. ORR = number of diseased dogs with complete response and partial response / total number of diseased dogs × 100%; CR = number of diseased dogs with all detectable tumors disappeared/ total number of diseased dogs × 100%; and PR = number of diseased dogs with sum of longest diameters of target lesions reduced by at least 30% / total number of diseased dogs × 100%.

The preliminary clinical therapeutic effects were shown in Table 5 below: both 3334 chimeric antibody and 3334-H1K2-canIgG B had better anti-tumor effect; and the therapeutic effect of 3334-H1K2-canIgG B was better than that of 3334 chimeric antibody.

**Table 5 Clinical anti-tumor efficacy data of 3334 and 3334-H1K2-canIgG B**

| Treatment group | ORR | | CR | |
|---|---|---|---|---|
| | Melanoma | Mast cell tumor | Melanoma | Mast cell tumor |
| 3334-canIgG B | 40% (4/10) | 30% (3/10) | 10% (1/10) | 0 |
| 3334-H1K2-canIgG B | 60% (6/10) | 50% (5/10) | 30% (3/10) | 10% (1/10) |

It can be seen from the above results that the antibodies obtained from screening by the present invention have high affinity and high activity, and can be used to treat canine tumor diseases by blocking the binding of canine PD-1 to its ligand.

Although the present invention has been described in detail with the general description and specific embodiments, some modifications or improvements can be made on the basis of the present invention, which would be apparent to those skilled in the art. Therefore, these modifications or improvements made without departing from the spirit of the present invention fall within the scope of protection of the present invention.

**Table of Sequences**

| SEQ ID NO: | Antibody | Description | Amino Acid/Nucleotide Sequence |
|---|---|---|---|
| 1 | - | PD-1 protein | MGSRRGPWPLVWAVLQLGWWPGWLLDSP DRPWSPLTFSPAQLTVQEGENATFTCSLADI PDSFVLNWYRLSPRNQTDKLAAFQEDRIEPG RDRRFRVTRLPNGRDFHMSIVAARLNDSGIY LCGAIYLPPNTQINESPRAELSVTERTLEPPT QSPSPPPRLSGQLQG |
| 2 | 3334 | VH-CDR1 | NYLIE |
| 3 | 3334 | VH-CDR2 | VINPRNGGTDYNEQFKG |
| 4 | 3334 | VH-CDR3 | GDYYDTGSLAMDY |
| 5 | 3334 | VL-CDR1 | RASKSVSTSAYSYMH |
| 6 | 3334 | VL-CDR2 | LASNLES |
| 7 | 3334 | VL-CDR3 | QHSRELPPT |
| 8 | 3334 | VH-CDR1 | GYAFTNY |
| 9 | 3334 | VH-CDR2 | NPRNGG |
| 10 | 3334 | VH-CDR2 | INPRNGGT |
| 11 | 3334 | VH-CDR3 | ARGDYYDTGSLAMDY |
| 12 | 3334 | VL-CDR1 | KSVSTSAYSY |
| 13 | 3334 | VL-CDR2 | LA |
| 14 | 3334 | VH-CDR1 | GYAFTNYL |
| 15 | 3334 | VL-CDR2 | LAS |
| 16 | 3334 | chimeric-VH | QVHVKQSGAELVRPGTSVKVSCKASGYAFT NYLIEWVKQRPGQGLEWIGVINPRNGGTDY NEQFKGRATLTADKSSSTAYMQLSSLTSEDS AVYFCARGDYYDTGSLAMDYWGQGTSVT VSS |
| 17 | 3334 | chimeric-VL | DIVLTQSPASLAVSLGQRATISCRASKSVSTS AYSYMHWYQQKPGQPPKLLIYLASNLESGV PARFSGSGSGTDFTLNIHPVEEEDAATYYCQ HSRELPPTFGGGTKLEIK |
| 18 | 3334, caninized | caninized H1-VH | EVQLVQSGAEVKKPGASVKVSCKTSGYAFT NYLIEWVRQAPGAGLDWMGVINPRNGGTD YNEQFKGRVTLTADKSTSTAYMELSSLRAG DIAVYYCARGDYYDTGSLAMDYWGQGTLV TVSS |
| 19 | 3334, caninized | caninized H2-VH | EVQLVESGGDLVKPGGSLRLSCVASGYAFT NYLIEWVRQAPGKGLQWVGVINPRNGGTD YNEQFKGRFTISADKAKNTAYLQMNSLRAE DTAVYYCARGDYYDTGSLAMDYWGQGTL VTVSS |
| 20 | 3334, caninized | caninized H3-VH | EVQLVESGGDLVKPGGSLRLSCVASGYAFT NYLIEWIRQAPGKGNQWVGVINPRNGGTDY NEQFKGRFTISADKAKNTAYLQMNSLTVED TALYYCARGDYYDTGSLAMDYWGQGTLV TVSS |
| 21 | 3334, caninized | caninized K1-VL | EIVLTQTPLSLAVTPGELATISCRASKSVSTS AYSYMHWYLQKPGQTPRLLIYLASNLESGV SDRFSGSGSGTDFTLKISRVEAEDVGVYYCQ HSRELPPTFGQGTKLEIK |
| 22 | 3334, caninized | caninized K2-VL | DIVLTQTPLSLSVSPGETASISCRASKSVSTSA YSYMHWYRQKPGQSPQLLIYLASNLESGVP DRFSGSGSGTDFTLRISRVEADDAGVYYCQ HSRELPPTFGQGTKLEIK |
| 23 | 3334, caninized | caninized K3-VL | DIVLTQTPLSLSVSPGEPASISCRASKSVSTSA YSYMHWYRQKPGQSPQLLIYLASNLESWVP DRFSGSGSGTDFTLRISRVEADDAGVYYCQ HSRELPPTFGQGTKLEIK |
| 24 | - | canine IgG2 CH | ASTTAPSVFPLAPSCGSTSGSTVALACLVSG YFPEPVTVSWNSGSLTSGVHTFPSVLQSSGL YSLSSMVTVPSSRWPSETFTCNVAHPASKTK VDKPVPKRENGRVPRPPDCPKCPAPEAAGA PSVFIFPPKPKDTLLIARTPEVTCVVVDLDPE DPEVQISWFVDGKQMQTAKTQPREEQFNGT YRVVSVLPIGHQDWLKGKQFTCKVNNKAL PSPIERTISKARGQAHQPSVYVLPPSREELSK NTVSLTCLIKDFFPPDIDVEWQSNGQQEPES KYRTTPPQLDEDGSYFLYSKLSVDKSRWQR GDTFICAVMHEALHNHYTQESLSHSPGK |
| 25 | - | Kappa CL | RNDAQPAVYLFQPSPDQLHTGSASVVCLLN SFYPKDINVKWKVDGVIQDTGIQESVTEQD KDSTYSLSSTLTMSSTEYLSHELYSCEITHKS LPSTLIKSFQRSEC |
| 26 | 3334 | chimeric-heavy chain full length | QVHVKQSGAELVRPGTSVKVSCKASGYAFT NYLIEWVKQRPGQGLEWIGVINPRNGGTDY NEQFKGRATLTADKSSSTAYMQLSSLTSEDS AVYFCARGDYYDTGSLAMDYWGQGTSVT VSSASTTAPSVFPLAPSCGSTSGSTVALACLV SGYFPEPVTVSWNSGSLTSGVHTFPSVLQSS GLYSLSSMVTVPSSRWPSETFTCNVAHPASK TKVDKPVPKRENGRVPRPPDCPKCPAPEAA GAPSVFIFPPKPKDTLLIARTPEVTCVVVDLD PEDPEVQISWFVDGKQMQTAKTQPREEQFN GTYRVVSVLPIGHQDWLKGKQFTCKVNNK ALPSPIERTISKARGQAHQPSVYVLPPSREEL SKNTVSLTCLIKDFFPPDIDVEWQSNGQQEP ESKYRTTPPQLDEDGSYFLYSKLSVDKSRW QRGDTFICAVMHEALHNHYTQESLSHSPGK |
| 27 | 3334 | chimeric-light chain full length | DIVLTQSPASLAVSLGQRATISCRASKSVSTS AYSYMHWYQQKPGQPPKLLIYLASNLESGV PARFSGSGSGTDFTLNIHPVEEEDAATYYCQ HSRELPPTFGGGTKLEIKRNDAQPAVYLFQP SPDQLHTGSASVVCLLNSFYPKDINVKWKV DGVIQDTGIQESVTEQDKDSTYSLSSTLTMS STEYLSHELYSCEITHKSLPSTLIKSFQRSEC |
| 28 | 3334, caninized | caninized H1-heavy chain full length | EVQLVQSGAEVKKPGASVKVSCKTSGYAFT NYLIEWVRQAPGAGLDWMGVINPRNGGTD YNEQFKGRVTLTADKSTSTAYMELSSLRAG DIAVYYCARGDYYDTGSLAMDYWGQGTLV TVSSASTTAPSVFPLAPSCGSTSGSTVALACL VSGYFPEPVTVSWNSGSLTSGVHTFPSVLQS SGLYSLSSMVTVPSSRWPSETFTCNVAHPAS KTKVDKPVPKRENGRVPRPPDCPKCPAPEA AGAPSVFIFPPKPKDTLLIARTPEVTCVVVDL DPEDPEVQISWFVDGKQMQTAKTQPREEQF NGTYRVVSVLPIGHQDWLKGKQFTCKVNN KALPSPIERTISKARGQAHQPSVYVLPPSREE LSKNTVSLTCLIKDFFPPDIDVEWQSNGQQE PESKYRTTPPQLDEDGSYFLYSKLSVDKSRW QRGDTFICAVMHEALHNHYTQESLSHSPGK |
| 29 | 3334, caninized | caninized H2-heavy chain full length | EVQLVESGGDLVKPGGSLRLSCVASGYAFT NYLIEWVRQAPGKGLQWVGVINPRNGGTD YNEQFKGRFTISADKAKNTAYLQMNSLRAE DTAVYYCARGDYYDTGSLAMDYWGQGTL VTVSSASTTAPSVFPLAPSCGSTSGSTVALAC LVSGYFPEPVTVSWNSGSLTSGVHTFPSVLQ SSGLYSLSSMVTVPSSRWPSETFTCNVAHPA SKTKVDKPVPKRENGRVPRPPDCPKCPAPE AAGAPSVFIFPPKPKDTLLIARTPEVTCVVVD LDPEDPEVQISWFVDGKQMQTAKTQPREEQ FNGTYRVVSVLPIGHQDWLKGKQFTCKVN NKALPSPIERTISKARGQAHQPSVYVLPPSRE ELSKNTVSLTCLIKDFFPPDIDVEWQSNGQQ EPESKYRTTPPQLDEDGSYFLYSKLSVDKSR WQRGDTFICAVMHEALHNHYTQESLSHSPG K |
| 30 | 3334, caninized | caninized H3-heavy chain full length | EVQLVESGGDLVKPGGSLRLSCVASGYAFT NYLIEWIRQAPGKGNQWVGVINPRNGGTDY NEQFKGRFTISADKAKNTAYLQMNSLTVED TALYYCARGDYYDTGSLAMDYWGQGTLV TVSSASTTAPSVFPLAPSCGSTSGSTVALACL VSGYFPEPVTVSWNSGSLTSGVHTFPSVLQS SGLYSLSSMVTVPSSRWPSETFTCNVAHPAS KTKVDKPVPKRENGRVPRPPDCPKCPAPEA AGAPSVFIFPPKPKDTLLIARTPEVTCVVVDL DPEDPEVQISWFVDGKQMQTAKTQPREEQF NGTYRVVSVLPIGHQDWLKGKQFTCKVNN KALPSPIERTISKARGQAHQPSVYVLPPSREE LSKNTVSLTCLIKDFFPPDIDVEWQSNGQQE PESKYRTTPPQLDEDGSYFLYSKLSVDKSRW QRGDTFICAVMHEALHNHYTQESLSHSPGK |
| 31 | 3334, caninized | caninized K1-light chain full length | EIVLTQTPLSLAVTPGELATISCRASKSVSTS AYSYMHWYLQKPGQTPRLLIYLASNLESGV SDRFSGSGSGTDFTLKISRVEAEDVGVYYCQ HSRELPPTFGQGTKLEIKRNDAQPAVYLFQP SPDQLHTGSASVVCLLNSFYPKDINVKWKV DGVIQDTGIQESVTEQDKDSTYSLSSTLTMS STEYLSHELYSCEITHKSLPSTLIKSFQRSEC |
| 32 | 3334, caninized | caninized K2-light chain full length | DIVLTQTPLSLSVSPGETASISCRASKSVSTSA YSYMHWYRQKPGQSPQLLIYLASNLESGVP DRFSGSGSGTDFTLRISRVEADDAGVYYCQ HSRELPPTFGQGTKLEIKRNDAQPAVYLFQP SPDQLHTGSASVVCLLNSFYPKDINVKWKV DGVIQDTGIQESVTEQDKDSTYSLSSTLTMS STEYLSHELYSCEITHKSLPSTLIKSFQRSEC |
| 33 | 3334, caninized | caninized K3-light chain full length | DIVLTQTPLSLSVSPGEPASISCRASKSVSTSA YSYMHWYRQKPGQSPQLLIYLASNLESWVP DRFSGSGSGTDFTLRISRVEADDAGVYYCQ HSRELPPTFGQGTKLEIKRNDAQPAVYLFQP SPDQLHTGSASVVCLLNSFYPKDINVKWKV DGVIQDTGIQESVTEQDKDSTYSLSSTLTMS STEYLSHELYSCEITHKSLPSTLIKSFQRSEC |
| 34 | - | PD-1 protein, nucleotide sequence | ATGGGTAGCCGTAGGGGCCCTTGGCCTTTA GTGTGGGCCGTGCTACAGTTGGGTTGGTG GCCCGGCTGGCTGTTGGATTCGCCGGATCG CCCTTGGTCACCCCTAACTTTCTCCCCAGC TCAATTAACCGTACAAGAAGGTGAGAATG CAACTTTCACTTGTAGTCTGGCAGATATCC CGGATAGTTTTGTGCTGAACTGGTACCGTC TTTCCCCGCGCAACCAAACTGACAAGCTG GCGGCTTTTCAAGAGGATCGCATTGAACCT GGGCGAGACAGACGGTTTCGGGTTACCCG GCTGCCGAATGGTCGAGACTTCCATATGTC GATTGTGGCAGCACGATTGAATGACAGTG GTATCTATCTGTGTGGCGCGATCTACTTACC TCCAAATACACAAATTAATGAAAGCCCTCG AGCGGAACTAAGCGTAACGGAAAGGACCC TCGAGCCACCGACCCAATCCCCTTCCCCGC CCCCACGTCTTAGTGGTCAACTGCAGGGC |
| 35 | 3334 | VH-CDR1, nucleotide sequence | AACTACCTGATCGAG |
| 36 | 3334 | VH-CDR2, nucleotide sequence | GTGATCAACCCTAGAAACGGCGGCACCGA CTACAACGAGCAGTTCAAGGGC |
| 37 | 3334 | VH-CDR3, nucleotide sequence | GGCGACTACTACGACACCGGCAGCCTGGC CATGGACTAC |
| 38 | 3334 | VL-CDR1, nucleotide sequence | AGAGCCAGCAAGAGCGTGAGCACCAGCG CCTACAGCTACATGCAC |
| 39 | 3334 | VL-CDR2, nucleotide sequence | CTGGCCAGCAACCTGGAGAGC |
| 40 | 3334 | VL-CDR3, nucleotide sequence | CAGCACAGCAGAGAGCTGCCTCCTACC |
| 41 | 3334 | VH-CDR1, nucleotide sequence | GGCTACGCCTTCACCAACTAC |
| 42 | 3334 | VH-CDR2, nucleotide sequence | AACCCTAGAAACGGCGGC |
| 43 | 3334 | VH-CDR2, nucleotide sequence | ATCAACCCTAGAAACGGCGGCACC |
| 44 | 3334 | VH-CDR3, nucleotide sequence | GCCAGAGGCGACTACTACGACACCGGCAG CCTGGCCATGGACTAC |
| 45 | 3334 | VL-CDR1, nucleotide sequence | AAGAGCGTGAGCACCAGCGCCTACAGCTA C |
| 46 | 3334 | VL-CDR2, nucleotide sequence | CTGGCC |
| 47 | 3334 | VH-CDR1, nucleotide sequence | GGCTACGCCTTCACCAACTACCTG |
| 48 | 3334 | VL-CDR2, nucleotide sequence | CTGGCCAGC |
| 49 | 3334 | chimeric-V H, nucleotide sequence | CAGGTGCACGTGAAGCAGAGCGGCGCCG AGCTGGTGAGACCTGGCACCAGCGTGAAG GTGAGCTGCAAGGCCAGCGGCTACGCCTT CACCAACTACCTGATCGAGTGGGTGAAGC AGAGACCTGGCCAGGGCCTGGAGTGGATC GGCGTGATCAACCCTAGAAACGGCGGCAC CGACTACAACGAGCAGTTCAAGGGCAGAG CCACCCTGACCGCCGACAAGAGCAGCAGC ACCGCCTACATGCAGCTGAGCAGCCTGAC CAGCGAGGACAGCGCCGTGTACTTCTGCG CCAGAGGCGACTACTACGACACCGGCAGC CTGGCCATGGACTACTGGGGCCAGGGCAC CAGCGTGACCGTGAGCAGC |
| 50 | 3334 | chimeric-V L, nucleotide sequence | GACATCGTGCTGACCCAGAGCCCTGCCAG CCTGGCCGTGAGCCTGGGCCAGAGAGCCA CCATCAGCTGCAGAGCCAGCAAGAGCGTG AGCACCAGCGCCTACAGCTACATGCACTG GTACCAGCAGAAGCCTGGCCAGCCTCCTA AGCTGCTGATCTACCTGGCCAGCAACCTG GAGAGCGGCGTGCCTGCCAGATTCAGCGG CAGCGGCAGCGGCACCGACTTCACCCTGA ACATCCACCCTGTGGAGGAGGAGGACGCC GCCACCTACTACTGCCAGCACAGCAGAGA GCTGCCTCCTACCTTCGGCGGCGGCACCA AGCTGGAGATCAAG |
| 51 | 3334, caninized | caninized H1-VH, nucleotide sequence | GAAGTGCAACTAGTGCAAAGTGGTGCAGA AGTCAAGAAGCCTGGAGCTTCCGTGAAAG TGTCCTGCAAGACCTCTGGCTACGCCTTCA CTAACTACCTGATCGAGTGGGTGCGGCAG GCTCCTGGCGCTGGACTGGACTGGATGGG CGTGATCAATCCCCGGAACGGCGGCACCG ACTACAACGAGCAGTTCAAGGGCAGAGTG ACCCTGACCGCTGATAAGTCCACATCCACC GCCTACATGGAACTGTCCAGCCTCAGAGC CGGCGACATCGCCGTGTACTACTGTGCCAG AGGCGATTATTACGACACCGGCTCTCTGGC CATGGACTACTGGGGCCAGGGCACACTGG TGACCGTGTCTAGC |
| 52 | 3334, caninized | caninized H2-VH, nucleotide sequence | GAAGTGCAACTAGTGGAAAGTGGTGGTGA TCTCGTGAAGCCCGGCGGAAGCCTGCGGC TGTCCTGCGTGGCCTCTGGCTACGCCTTCA CTAACTACCTGATCGAGTGGGTGCGGCAA GCTCCTGGCAAGGGCCTGCAGTGGGTCGG CGTGATCAACCCTAGAAATGGCGGCACCG ACTACAACGAGCAGTTTAAAGGCAGATTC ACCATCAGCGCCGACAAGGCCAAGAACAC CGCTTATCTGCAGATGAACTCCCTGCGCGC TGAAGATACCGCCGTGTACTACTGTGCTAG AGGCGATTACTACGACACAGGATCTCTGGC CATGGACTACTGGGGCCAGGGCACCCTGG TGACCGTGTCCTCT |
| 53 | 3334, caninized | caninized H3-VH, nucleotide sequence | GAAGTGCAACTAGTGGAAAGTGGTGGTGA TCTCGTGAAGCCCGGAGGAAGCCTGAGAC TGTCCTGCGTGGCCTCTGGCTACGCCTTCA CTAACTACCTGATCGAGTGGATCCGGCAAG CTCCTGGCAAAGGCAACCAGTGGGTCGGC GTGATCAATCCTCGGAACGGCGGCACCGA CTACAACGAGCAGTTCAAGGGCAGATTTA CCATCAGCGCCGACAAGGCCAAGAACACC GCCTACCTGCAGATGAACTCCCTGACCGTG GAAGATACAGCTCTGTACTACTGTGCTAGA GGCGATTATTACGACACCGGCTCTCTGGCC ATGGACTACTGGGGCCAGGGCACACTGGT GACCGTGTCCTCT |
| 54 | 3334, caninized | caninized K1-VL, nucleotide sequence | GAGATCGTGCTGACCCAGACCCCCCTGAG CCTGGCCGTGACCCCCGGCGAGCTGGCCA CCATCAGCTGCCGGGCCAGCAAGAGCGTG AGCACCAGCGCCTACAGCTACATGCACTG GTACCTGCAGAAGCCCGGCCAGACCCCCC GGCTGCTGATCTACCTGGCCAGCAACCTG GAGAGCGGCGTGAGCGACCGGTTCAGCGG CAGCGGCAGCGGCACCGACTTCACCCTGA AGATCAGCCGGGTGGAGGCCGAGGACGTG GGCGTGTACTACTGCCAGCACAGCCGGGA GCTGCCCCCCACCTTCGGCCAGGGCACCA AGCTGGAGATCAAG |
| 55 | 3334, caninize d | caninized K2-VL, nucleotide sequence | GACATCGTGCTGACCCAGACCCCCCTGAG CCTGAGCGTGAGCCCCGGCGAGACCGCCA GCATCAGCTGCCGGGCCAGCAAGAGCGTG AGCACCAGCGCCTACAGCTACATGCACTG GTACCGGCAGAAGCCCGGCCAGAGCCCCC AGCTGCTGATCTACCTGGCCAGCAACCTG GAGAGCGGCGTGCCCGACCGGTTCAGCGG CAGCGGCAGCGGCACCGACTTCACCCTGC GGATCAGCCGGGTGGAGGCCGACGACGCC GGCGTGTACTACTGCCAGCACAGCCGGGA GCTGCCCCCCACCTTCGGCCAGGGCACCA AGCTGGAGATCAAG |
| 56 | 3334, caninized | caninized K3-VL, nucleotide sequence | GACATCGTGCTGACCCAGACCCCCCTGAG CCTGAGCGTGAGCCCCGGCGAGCCCGCCA GCATCAGCTGCCGGGCCAGCAAGAGCGTG AGCACCAGCGCCTACAGCTACATGCACTG GTACCGGCAGAAGCCCGGCCAGAGCCCCC AGCTGCTGATCTACCTGGCCAGCAACCTG GAGAGCTGGGTGCCCGACCGGTTCAGCGG CAGCGGCAGCGGCACCGACTTCACCCTGC GGATCAGCCGGGTGGAGGCCGACGACGCC GGCGTGTACTACTGCCAGCACAGCCGGGA GCTGCCCCCCACCTTCGGCCAGGGCACCA AGCTGGAGATCAAG |
| 57 | - | canine IgG2 CH, nucleotide sequence | GCCAGCACCACCGCCCCCAGCGTGTTCCC CCTGGCCCCCAGCTGCGGCAGCACCAGCG GCAGCACCGTGGCCCTGGCCTGCCTGGTG AGCGGCTACTTCCCCGAGCCCGTGACCGT GAGCTGGAACAGCGGCAGCCTGACCAGCG GCGTGCACACCTTCCCCAGCGTGCTGCAG AGCAGCGGCCTGTACAGCCTGAGCAGCAT GGTGACCGTGCCCAGCAGCCGGTGGCCCA GCGAGACCTTCACCTGCAACGTGGCCCAC CCCGCCAGCAAGACCAAGGTGGACAAGCC CGTGCCCAAGCGGGAGAACGGCCGGGTGC CCCGGCCCCCCGACTGCCCCAAGTGCCCC GCCCCCGAGGCCGCCGGCGCCCCCAGCGT GTTCATCTTCCCCCCCAAGCCCAAGGACAC CCTGCTGATCGCCCGGACCCCCGAGGTGA CCTGCGTGGTGGTGGACCTGGACCCCGAG GACCCCGAGGTGCAGATCAGCTGGTTCGT GGACGGCAAGCAGATGCAGACCGCCAAG ACCCAGCCCCGGGAGGAGCAGTTCAACGG CACCTACCGGGTGGTGAGCGTGCTGCCCA TCGGCCACCAGGACTGGCTGAAGGGCAAG CAGTTCACCTGCAAGGTGAACAACAAGGC CCTGCCCAGCCCCATCGAGCGGACCATCA GCAAGGCCCGGGGCCAGGCCCACCAGCCC AGCGTGTACGTGCTGCCCCCCAGCCGGGA GGAGCTGAGCAAGAACACCGTGAGCCTGA CCTGCCTGATCAAGGACTTCTTCCCCCCCG ACATCGACGTGGAGTGGCAGAGCAACGGC CAGCAGGAGCCCGAGAGCAAGTACCGGA CCACCCCCCCCCAGCTGGACGAGGACGGC AGCTACTTCCTGTACAGCAAGCTGAGCGT GGACAAGAGCCGGTGGCAGCGGGGCGAC ACCTTCATCTGCGCCGTGATGCACGAGGCC CTGCACAACCACTACACCCAGGAGAGCCT GAGCCACAGCCCCGGCAAG |
| 58 | - | Kappa CL, nucleotide sequence | CGGAACGACGCCCAGCCCGCCGTGTACCT GTTCCAGCCCAGCCCCGACCAGCTGCACA CCGGCAGCGCCAGCGTGGTGTGCCTGCTG AACAGCTTCTACCCCAAGGACATCAACGT GAAGTGGAAGGTGGACGGCGTGATCCAGG ACACCGGCATCCAGGAGAGCGTGACCGAG CAGGACAAGGACAGCACCTACAGCCTGAG CAGCACCCTGACCATGAGCAGCACCGAGT ACCTGAGCCACGAGCTGTACAGCTGCGAG ATCACCCACAAGAGCCTGCCCAGCACCCT GATCAAGAGCTTCCAGCGGAGCGAGTGC |
| 59 | 3334 | chimeric-heavy chain full length, nucleotide sequence | CAGGTGCACGTGAAGCAGAGCGGCGCCG AGCTGGTGAGACCTGGCACCAGCGTGAAG GTGAGCTGCAAGGCCAGCGGCTACGCCTT CACCAACTACCTGATCGAGTGGGTGAAGC AGAGACCTGGCCAGGGCCTGGAGTGGATC GGCGTGATCAACCCTAGAAACGGCGGCAC CGACTACAACGAGCAGTTCAAGGGCAGAG CCACCCTGACCGCCGACAAGAGCAGCAGC ACCGCCTACATGCAGCTGAGCAGCCTGAC CAGCGAGGACAGCGCCGTGTACTTCTGCG CCAGAGGCGACTACTACGACACCGGCAGC CTGGCCATGGACTACTGGGGCCAGGGCAC CAGCGTGACCGTGAGCAGCGCCAGCACCA CCGCCCCCAGCGTGTTCCCCCTGGCCCCCA GCTGCGGCAGCACCAGCGGCAGCACCGTG GCCCTGGCCTGCCTGGTGAGCGGCTACTTC CCCGAGCCCGTGACCGTGAGCTGGAACAG CGGCAGCCTGACCAGCGGCGTGCACACCT TCCCCAGCGTGCTGCAGAGCAGCGGCCTG TACAGCCTGAGCAGCATGGTGACCGTGCC CAGCAGCCGGTGGCCCAGCGAGACCTTCA CCTGCAACGTGGCCCACCCCGCCAGCAAG ACCAAGGTGGACAAGCCCGTGCCCAAGCG GGAGAACGGCCGGGTGCCCCGGCCCCCCG ACTGCCCCAAGTGCCCCGCCCCCGAGGCC GCCGGCGCCCCCAGCGTGTTCATCTTCCCC CCCAAGCCCAAGGACACCCTGCTGATCGC CCGGACCCCCGAGGTGACCTGCGTGGTGG TGGACCTGGACCCCGAGGACCCCGAGGTG CAGATCAGCTGGTTCGTGGACGGCAAGCA GATGCAGACCGCCAAGACCCAGCCCCGGG AGGAGCAGTTCAACGGCACCTACCGGGTG GTGAGCGTGCTGCCCATCGGCCACCAGGA CTGGCTGAAGGGCAAGCAGTTCACCTGCA AGGTGAACAACAAGGCCCTGCCCAGCCCC ATCGAGCGGACCATCAGCAAGGCCCGGGG CCAGGCCCACCAGCCCAGCGTGTACGTGC TGCCCCCCAGCCGGGAGGAGCTGAGCAAG AACACCGTGAGCCTGACCTGCCTGATCAA GGACTTCTTCCCCCCCGACATCGACGTGGA GTGGCAGAGCAACGGCCAGCAGGAGCCC GAGAGCAAGTACCGGACCACCCCCCCCCA GCTGGACGAGGACGGCAGCTACTTCCTGT ACAGCAAGCTGAGCGTGGACAAGAGCCG GTGGCAGCGGGGCGACACCTTCATCTGCG CCGTGATGCACGAGGCCCTGCACAACCAC TACACCCAGGAGAGCCTGAGCCACAGCCC CGGCAAG |
| 60 | 3334 | chimeric-light chain full length, nucleotide sequence | GACATCGTGCTGACCCAGAGCCCTGCCAG CCTGGCCGTGAGCCTGGGCCAGAGAGCCA CCATCAGCTGCAGAGCCAGCAAGAGCGTG AGCACCAGCGCCTACAGCTACATGCACTG GTACCAGCAGAAGCCTGGCCAGCCTCCTA AGCTGCTGATCTACCTGGCCAGCAACCTG GAGAGCGGCGTGCCTGCCAGATTCAGCGG CAGCGGCAGCGGCACCGACTTCACCCTGA ACATCCACCCTGTGGAGGAGGAGGACGCC GCCACCTACTACTGCCAGCACAGCAGAGA GCTGCCTCCTACCTTCGGCGGCGGCACCA AGCTGGAGATCAAGCGGAACGACGCCCAG CCCGCCGTGTACCTGTTCCAGCCCAGCCCC GACCAGCTGCACACCGGCAGCGCCAGCGT GGTGTGCCTGCTGAACAGCTTCTACCCCA AGGACATCAACGTGAAGTGGAAGGTGGAC GGCGTGATCCAGGACACCGGCATCCAGGA GAGCGTGACCGAGCAGGACAAGGACAGC ACCTACAGCCTGAGCAGCACCCTGACCAT GAGCAGCACCGAGTACCTGAGCCACGAGC TGTACAGCTGCGAGATCACCCACAAGAGC CTGCCCAGCACCCTGATCAAGAGCTTCCA GCGGAGCGAGTGC |
| 61 | 3334, caninized | caninized H1-heavy chain full length, nucleotide sequence | GAAGTGCAACTAGTGCAAAGTGGTGCAGA AGTCAAGAAGCCTGGAGCTTCCGTGAAAG TGTCCTGCAAGACCTCTGGCTACGCCTTCA CTAACTACCTGATCGAGTGGGTGCGGCAG GCTCCTGGCGCTGGACTGGACTGGATGGG CGTGATCAATCCCCGGAACGGCGGCACCG ACTACAACGAGCAGTTCAAGGGCAGAGTG ACCCTGACCGCTGATAAGTCCACATCCACC GCCTACATGGAACTGTCCAGCCTCAGAGC CGGCGACATCGCCGTGTACTACTGTGCCAG AGGCGATTATTACGACACCGGCTCTCTGGC CATGGACTACTGGGGCCAGGGCACACTGG TGACCGTGTCTAGCGCCAGCACCACCGCC CCCAGCGTGTTCCCCCTGGCCCCCAGCTGC GGCAGCACCAGCGGCAGCACCGTGGCCCT GGCCTGCCTGGTGAGCGGCTACTTCCCCG AGCCCGTGACCGTGAGCTGGAACAGCGGC AGCCTGACCAGCGGCGTGCACACCTTCCC CAGCGTGCTGCAGAGCAGCGGCCTGTACA GCCTGAGCAGCATGGTGACCGTGCCCAGC AGCCGGTGGCCCAGCGAGACCTTCACCTG CAACGTGGCCCACCCCGCCAGCAAGACCA AGGTGGACAAGCCCGTGCCCAAGCGGGA GAACGGCCGGGTGCCCCGGCCCCCCGACT GCCCCAAGTGCCCCGCCCCCGAGGCCGCC GGCGCCCCCAGCGTGTTCATCTTCCCCCCC AAGCCCAAGGACACCCTGCTGATCGCCCG GACCCCCGAGGTGACCTGCGTGGTGGTGG ACCTGGACCCCGAGGACCCCGAGGTGCAG ATCAGCTGGTTCGTGGACGGCAAGCAGAT GCAGACCGCCAAGACCCAGCCCCGGGAG GAGCAGTTCAACGGCACCTACCGGGTGGT GAGCGTGCTGCCCATCGGCCACCAGGACT GGCTGAAGGGCAAGCAGTTCACCTGCAAG GTGAACAACAAGGCCCTGCCCAGCCCCAT CGAGCGGACCATCAGCAAGGCCCGGGGCC AGGCCCACCAGCCCAGCGTGTACGTGCTG CCCCCCAGCCGGGAGGAGCTGAGCAAGA ACACCGTGAGCCTGACCTGCCTGATCAAG GACTTCTTCCCCCCCGACATCGACGTGGAG TGGCAGAGCAACGGCCAGCAGGAGCCCG AGAGCAAGTACCGGACCACCCCCCCCAG CTGGACGAGGACGGCAGCTACTTCCTGTA CAGCAAGCTGAGCGTGGACAAGAGCCGGT GGCAGCGGGGCGACACCTTCATCTGCGCC GTGATGCACGAGGCCCTGCACAACCACTA CACCCAGGAGAGCCTGAGCCACAGCCCCG GCAAG |
| 62 | 3334, caninized | caninized H2-heavy chain full length, nucleotide sequence | GAAGTGCAACTAGTGGAAAGTGGTGGTGA TCTCGTGAAGCCCGGCGGAAGCCTGCGGC TGTCCTGCGTGGCCTCTGGCTACGCCTTCA CTAACTACCTGATCGAGTGGGTGCGGCAA GCTCCTGGCAAGGGCCTGCAGTGGGTCGG CGTGATCAACCCTAGAAATGGCGGCACCG ACTACAACGAGCAGTTTAAAGGCAGATTC ACCATCAGCGCCGACAAGGCCAAGAACAC CGCTTATCTGCAGATGAACTCCCTGCGCGC TGAAGATACCGCCGTGTACTACTGTGCTAG AGGCGATTACTACGACACAGGATCTCTGGC CATGGACTACTGGGGCCAGGGCACCCTGG TGACCGTGTCCTCTGCCAGCACCACCGCC CCCAGCGTGTTCCCCCTGGCCCCCAGCTGC GGCAGCACCAGCGGCAGCACCGTGGCCCT GGCCTGCCTGGTGAGCGGCTACTTCCCCG AGCCCGTGACCGTGAGCTGGAACAGCGGC AGCCTGACCAGCGGCGTGCACACCTTCCC CAGCGTGCTGCAGAGCAGCGGCCTGTACA GCCTGAGCAGCATGGTGACCGTGCCCAGC AGCCGGTGGCCCAGCGAGACCTTCACCTG CAACGTGGCCCACCCCGCCAGCAAGACCA AGGTGGACAAGCCCGTGCCCAAGCGGGA GAACGGCCGGGTGCCCCGGCCCCCCGACT GCCCCAAGTGCCCCGCCCCCGAGGCCGCC GGCGCCCCCAGCGTGTTCATCTTCCCCCCC AAGCCCAAGGACACCCTGCTGATCGCCCG GACCCCCGAGGTGACCTGCGTGGTGGTGG ACCTGGACCCCGAGGACCCCGAGGTGCAG ATCAGCTGGTTCGTGGACGGCAAGCAGAT GCAGACCGCCAAGACCCAGCCCCGGGAG GAGCAGTTCAACGGCACCTACCGGGTGGT GAGCGTGCTGCCCATCGGCCACCAGGACT GGCTGAAGGGCAAGCAGTTCACCTGCAAG GTGAACAACAAGGCCCTGCCCAGCCCCAT CGAGCGGACCATCAGCAAGGCCCGGGGCC AGGCCCACCAGCCCAGCGTGTACGTGCTG CCCCCCAGCCGGGAGGAGCTGAGCAAGA ACACCGTGAGCCTGACCTGCCTGATCAAG GACTTCTTCCCCCCCGACATCGACGTGGAG TGGCAGAGCAACGGCCAGCAGGAGCCCG AGAGCAAGTACCGGACCACCCCCCCCCAG CTGGACGAGGACGGCAGCTACTTCCTGTA CAGCAAGCTGAGCGTGGACAAGAGCCGGT GGCAGCGGGGCGACACCTTCATCTGCGCC GTGATGCACGAGGCCCTGCACAACCACTA CACCCAGGAGAGCCTGAGCCACAGCCCCG GCAAG |
| 63 | 3334, caninized | caninized H3-heavy chain full length, nucleotide sequence | GAAGTGCAACTAGTGGAAAGTGGTGGTGA TCTCGTGAAGCCCGGAGGAAGCCTGAGAC TGTCCTGCGTGGCCTCTGGCTACGCCTTCA CTAACTACCTGATCGAGTGGATCCGGCAAG CTCCTGGCAAAGGCAACCAGTGGGTCGGC GTGATCAATCCTCGGAACGGCGGCACCGA CTACAACGAGCAGTTCAAGGGCAGATTTA CCATCAGCGCCGACAAGGCCAAGAACACC GCCTACCTGCAGATGAACTCCCTGACCGTG GAAGATACAGCTCTGTACTACTGTGCTAGA GGCGATTATTACGACACCGGCTCTCTGGCC ATGGACTACTGGGGCCAGGGCACACTGGT GACCGTGTCCTCTGCCAGCACCACCGCCC CCAGCGTGTTCCCCCTGGCCCCCAGCTGC GGCAGCACCAGCGGCAGCACCGTGGCCCT GGCCTGCCTGGTGAGCGGCTACTTCCCCG AGCCCGTGACCGTGAGCTGGAACAGCGGC AGCCTGACCAGCGGCGTGCACACCTTCCC CAGCGTGCTGCAGAGCAGCGGCCTGTACA GCCTGAGCAGCATGGTGACCGTGCCCAGC AGCCGGTGGCCCAGCGAGACCTTCACCTG CAACGTGGCCCACCCCGCCAGCAAGACCA AGGTGGACAAGCCCGTGCCCAAGCGGGA GAACGGCCGGGTGCCCCGGCCCCCCGACT GCCCCAAGTGCCCCGCCCCCGAGGCCGCC GGCGCCCCCAGCGTGTTCATCTTCCCCCCC AAGCCCAAGGACACCCTGCTGATCGCCCG GACCCCCGAGGTGACCTGCGTGGTGGTGG ACCTGGACCCCGAGGACCCCGAGGTGCAG ATCAGCTGGTTCGTGGACGGCAAGCAGAT GCAGACCGCCAAGACCCAGCCCCGGGAG GAGCAGTTCAACGGCACCTACCGGGTGGT GAGCGTGCTGCCCATCGGCCACCAGGACT GGCTGAAGGGCAAGCAGTTCACCTGCAAG GTGAACAACAAGGCCCTGCCCAGCCCCAT CGAGCGGACCATCAGCAAGGCCCGGGCC AGGCCCACCAGCCCAGCGTGTACGTGCTG CCCCCCAGCCGGGAGGAGCTGAGCAAGA ACACCGTGAGCCTGACCTGCCTGATCAAG GACTTCTTCCCCCCCGACATCGACGTGGAG TGGCAGAGCAACGGCCAGCAGGAGCCCG AGAGCAAGTACCGGACCACCCCCCCCCAG CTGGACGAGGACGGCAGCTACTTCCTGTA CAGCAAGCTGAGCGTGGACAAGAGCCGGT GGCAGCGGGGCGACACCTTCATCTGCGCC GTGATGCACGAGGCCCTGCACAACCACTA CACCCAGGAGAGCCTGAGCCACAGCCCCG GCAAG |
| 64 | 3334, caninized | caninized K1-light chain full length, nucleotide sequence | GAGATCGTGCTGACCCAGACCCCCCTGAG CCTGGCCGTGACCCCCGGCGAGCTGGCCA CCATCAGCTGCCGGGCCAGCAAGAGCGTG AGCACCAGCGCCTACAGCTACATGCACTG GTACCTGCAGAAGCCCGGCCAGACCCCC GGCTGCTGATCTACCTGGCCAGCAACCTG GAGAGCGGCGTGAGCGACCGGTTCAGCGG CAGCGGCAGCGGCACCGACTTCACCCTGA AGATCAGCCGGGTGGAGGCCGAGGACGTG GGCGTGTACTACTGCCAGCACAGCCGGGA GCTGCCCCCCACCTTCGGCCAGGGCACCA AGCTGGAGATCAAGCGGAACGACGCCCAG CCCGCCGTGTACCTGTTCCAGCCCAGCCCC GACCAGCTGCACACCGGCAGCGCCAGCGT GGTGTGCCTGCTGAACAGCTTCTACCCCA AGGACATCAACGTGAAGTGGAAGGTGGAC GGCGTGATCCAGGACACCGGCATCCAGGA GAGCGTGACCGAGCAGGACAAGGACAGC ACCTACAGCCTGAGCAGCACCCTGACCAT GAGCAGCACCGAGTACCTGAGCCACGAGC TGTACAGCTGCGAGATCACCCACAAGAGC CTGCCCAGCACCCTGATCAAGAGCTTCCA GCGGAGCGAGTGC |
| 65 | 3334, caninized | caninized K2-light chain full length, nucleotide sequence | GACATCGTGCTGACCCAGACCCCCCTGAG CCTGAGCGTGAGCCCCGGCGAGACCGCCA GCATCAGCTGCCGGGCCAGCAAGAGCGTG AGCACCAGCGCCTACAGCTACATGCACTG GTACCGGCAGAAGCCCGGCCAGAGCCCCC AGCTGCTGATCTACCTGGCCAGCAACCTG GAGAGCGGCGTGCCCGACCGGTTCAGCGG CAGCGGCAGCGGCACCGACTTCACCCTGC GGATCAGCCGGGTGGAGGCCGACGACGCC GGCGTGTACTACTGCCAGCACAGCCGGGA GCTGCCCCCCACCTTCGGCCAGGGCACCA AGCTGGAGATCAAGCGGAACGACGCCCAG CCCGCCGTGTACCTGTTCCAGCCCAGCCCC GACCAGCTGCACACCGGCAGCGCCAGCGT GGTGTGCCTGCTGAACAGCTTCTACCCCA AGGACATCAACGTGAAGTGGAAGGTGGAC GGCGTGATCCAGGACACCGGCATCCAGGA GAGCGTGACCGAGCAGGACAAGGACAGC ACCTACAGCCTGAGCAGCACCCTGACCAT GAGCAGCACCGAGTACCTGAGCCACGAGC TGTACAGCTGCGAGATCACCCACAAGAGC CTGCCCAGCACCCTGATCAAGAGCTTCCA GCGGAGCGAGTGC |
| 66 | 3334, caninized | caninized K3-light chain full length, nucleotide sequence | GACATCGTGCTGACCCAGACCCCCCTGAG CCTGAGCGTGAGCCCCGGCGAGCCCGCCA GCATCAGCTGCCGGGCCAGCAAGAGCGTG AGCACCAGCGCCTACAGCTACATGCACTG GTACCGGCAGAAGCCCGGCCAGAGCCCCC AGCTGCTGATCTACCTGGCCAGCAACCTG GAGAGCTGGGTGCCCGACCGGTTCAGCGG CAGCGGCAGCGGCACCGACTTCACCCTGC GGATCAGCCGGGTGGAGGCCGACGACGCC GGCGTGTACTACTGCCAGCACAGCCGGGA GCTGCCCCCCACCTTCGGCCAGGGCACCA AGCTGGAGATCAAGCGGAACGACGCCCAG CCCGCCGTGTACCTGTTCCAGCCCAGCCCC GACCAGCTGCACACCGGCAGCGCCAGCGT GGTGTGCCTGCTGAACAGCTTCTACCCCA AGGACATCAACGTGAAGTGGAAGGTGGAC GGCGTGATCCAGGACACCGGCATCCAGGA GAGCGTGACCGAGCAGGACAAGGACAGC ACCTACAGCCTGAGCAGCACCCTGACCAT GAGCAGCACCGAGTACCTGAGCCACGAGC TGTACAGCTGCGAGATCACCCACAAGAGC CTGCCCAGCACCCTGATCAAGAGCTTCCA GCGGAGCGAGTGC |

## Claims

1. An anti-PD-1 antibody or antigen-binding fragment, **characterized in that** the anti-PD-1 antibody or antigen-binding fragment is capable of binding to canine PD-1, and comprises CDRs 1, 2 and 3 of a heavy chain variable region and/or CDRs 1, 2 and 3 of a light chain variable region; wherein the antibody or antigen-binding fragment comprises an amino acid sequence having at least 85% identity to a CDR sequence selected from at least one of the following CDR sequences: CDR sequences of the heavy chain variable region selected from SEQ ID NOs: 2-4, 8-11 and 14, and CDR sequences of the light chain variable region selected from SEQ ID NOs: 5-7, 12-13 and 15.

2. The antibody or antigen-binding fragment according to claim 1, **characterized in that** the antibody or antigen-binding fragment comprises a CDR sequence selected from at least one of the following CDR sequences: CDR sequences of the heavy chain variable region selected from SEQ ID NOs: 2-4, 8-11 and 14, and CDR sequences of the light chain variable region selected from SEQ ID NOs: 5-7, 12-13 and 15.

3. An anti-PD-1 antibody or antigen-binding fragment, **characterized in that** the anti-PD-1 antibody or antigen-binding fragment is capable of binding to canine PD-1, and comprises CDRs 1, 2 and 3 of a heavy chain variable region and/or CDRs 1, 2 and 3 of a light chain variable region; and the heavy chain variable region is selected from SEQ ID NOs: 16 and 18-20, and the light chain variable region is selected from SEQ ID NOs: 17 and 21-23.

4. The antibody or antigen-binding fragment according to claim 1, **characterized in that** the antibody or antigen-binding fragment comprises a heavy chain variable region and/or a light chain variable region, wherein
the heavy chain variable region comprises CDR 1 shown in SEQ ID NO: 2, CDR 2 shown in SEQ ID NO: 3 and CDR 3 shown in SEQ ID NO: 4, the light chain variable region comprises CDR 1 shown in SEQ ID NO: 5, CDR 2 shown in SEQ ID NO: 6 and CDR 3 shown in SEQ ID NO: 7; or
the heavy chain variable region comprises CDR 1 shown in SEQ ID NO: 8, CDR 2 shown in SEQ ID NO: 9 and CDR 3 shown in SEQ ID NO: 4, the light chain variable region comprises CDR 1 shown in SEQ ID NO: 5, CDR 2 shown in SEQ ID NO: 6 and CDR 3 shown in SEQ ID NO: 7; or
the heavy chain variable region comprises CDR 1 shown in SEQ ID NO: 14, CDR 2 shown in SEQ ID NO: 10 and CDR 3 shown in SEQ ID NO: 11, the light chain variable region comprises CDR 1 shown in SEQ ID NO: 12, CDR 2 shown in SEQ ID NO: 13 or 15 and CDR 3 shown in SEQ ID NO: 7.

5. The antibody or antigen-binding fragment according to claim 1, **characterized in that** the antibody or antigen-binding fragment comprises a heavy chain variable region and/or a light chain variable region as follows: a heavy chain variable region shown in SEQ ID NO: 16 and/or a light chain variable region shown in SEQ ID NO: 17;
or a heavy chain variable region shown in SEQ ID NO: 18 and/or a light chain variable region shown in SEQ ID NO: 21;
or a heavy chain variable region shown in SEQ ID NO: 18 and/or a light chain variable region shown in SEQ ID NO: 22;
or a heavy chain variable region shown in SEQ ID NO: 18 and/or a light chain variable region shown in SEQ ID NO: 23;
or a heavy chain variable region shown in SEQ ID NO: 19 and/or a light chain variable region shown in SEQ ID NO: 21;
or a heavy chain variable region shown in SEQ ID NO: 19 and/or a light chain variable region shown in SEQ ID NO: 22;
or a heavy chain variable region shown in SEQ ID NO: 19 and/or a light chain variable region shown in SEQ ID NO: 23;
or a heavy chain variable region shown in SEQ ID NO: 20 and/or a light chain variable region shown in SEQ ID NO: 21;
or a heavy chain variable region shown in SEQ ID NO: 20 and/or a light chain variable region shown in SEQ ID NO: 22;
or a heavy chain variable region shown in SEQ ID NO: 20 and/or a light chain variable region shown in SEQ ID NO: 23.

6. The antibody or antigen-binding fragment according to claim 1, **characterized in that** a heavy chain constant region of the antibody or antigen-binding fragment is a heavy chain constant region of a canine IgG B mutant, with an amino acid sequence shown in SEQ ID NO: 24; and a light chain constant region of the antibody or antigen-binding fragment is a canine kappa chain constant region domain with an amino acid sequence shown in SEQ ID NO: 25.

7. The antibody or antigen-binding fragment according to claim 1, **characterized in that** the antibody or antigen-binding fragment comprises a full length of a heavy chain and/or a full length of a light chain, the sequence of the full length of the heavy chain used in the antibody or antigen-binding fragment is selected from any of SEQ ID NOs: 26 and 28-30, and the full length of the light chain used in the antibody or antigen-binding fragment is selected from any of SEQ ID NOs: 27 and 31-33.

8. The antibody or antigen-binding fragment according to claim 1, **characterized in that** in the antibody or antigen-binding fragment, the full length of the heavy chain is shown in SEQ ID NO: 26, and the full length of the light chain is shown in SEQ ID NO: 27;
or the full length of the heavy chain is shown in SEQ ID NO: 28, and the full length of the light chain is shown in SEQ ID NO: 31;
or the full length of the heavy chain is shown in SEQ ID NO: 28, and the full length of the light chain is shown in SEQ ID NO: 32;
or the full length of the heavy chain is shown in SEQ ID NO: 28, and the full length of the light chain is shown in SEQ ID NO: 33;
or the full length of the heavy chain is shown in SEQ ID NO: 29, and the full length of the light chain is shown in SEQ ID NO: 31;
or the full length of the heavy chain is shown in SEQ ID NO: 29, and the full length of the light chain is shown in SEQ ID NO: 32;
or the full length of the heavy chain is shown in SEQ ID NO: 29, and the full length of the light chain is shown in SEQ ID NO: 33;
or the full length of the heavy chain is shown in SEQ ID NO: 30, and the full length of the light chain is shown in SEQ ID NO: 31;
or the full length of the heavy chain is shown in SEQ ID NO: 30, and the full length of the light chain is shown in SEQ ID NO: 32;
or the full length of the heavy chain is shown in SEQ ID NO: 30, and the full length of the light chain is shown in SEQ ID NO: 33.

9. A nucleic acid molecule, **characterized in that** the nucleic acid molecule encodes the antibody or antigen-binding fragment thereof according to any of claims 1-8, and the nucleic acid molecule is a DNA or an RNA.

10. An expression vector, **characterized in that** a nucleotide sequence of the expression vector comprises the nucleic acid molecule according to claim 9.

11. A recombinant cell, **characterized in that** the recombinant cell is obtained by introducing the expression vector according to claim 10 into a host cell.

12. A pharmaceutical composition, **characterized in that** the pharmaceutical composition comprises the antibody or antigen-binding fragment according to any of claims 1-8, or the nucleic acid molecule according to claim 9, or the expression vector according to claim 10, or the recombinant cell according to claim 11.

13. Use of the antibody or antigen-binding fragment according to any of claims 1-8, or the nucleic acid molecule according to claim 9, or the expression vector according to claim 10, or the recombinant cell according to claim 11, or the pharmaceutical composition according to claim 12 in preventing and/or treating mammalian tumors, or in preparing a medicament for preventing and/or treating mammalian tumors, or in detecting PD-1 protein.
